# EUROPÄISCHE PATENTSCHRIFT

(11) **EP 1 314 754 B1**
(45) Veröffentlichungstag und Bekanntmachung des Hinweises auf die Patenterteilung: **22.09.2004**
(21) Anmeldenummer: 02025570.9
(22) Anmeldetag: 15.11.2002
(51) Int. Cl.: C08K 5/34, C08J 3/20, C09B 57/12, C09B 25/00

(54) **Verbrückte Perinone/Chinophthalone**
Bridged Perinones/Quinophtalones
Perinones/Quinophtalones Pontées

(30) Priorität: 27.11.2001 DE 10158137
(43) Veröffentlichungstag der Anmeldung: 28.05.2003
(73) Patentinhaber: Bayer Chemicals AG, 51368 Leverkusen (DE)
(72) Erfinder: Thiebes, Christoph, Dr., 50670 Köln (DE); Stawitz, Josef-Walter, Dr., 51519 Odenthal (DE); Feldhues, Ulrich, Dr., 51465 Bergisch Gladbach (DE)

(56) Entgegenhaltungen:
- EP-A- 0 827 986
- MANUKIAN B K ET AL: "CHINOPHTHALONE" CHIMIA, AARAU, CH, Bd. 24, Nr. 9, 1970, Seiten 328-339, XP002073520 ISSN: 0009-4293
- SHIGENORI OTSUKA ET AL: "A NEW DOUBLE LAYER PHOTOCONDUCTOR SYSTEM WITH ADVANCED RELIABILITY USING PERINONE BISAZOPIGMENT IN THE CARRIER GENERATION LAYER" PROCEEDINGS OF THE INTERNATIONAL CONGRESS ON ADVANCES IN NON IMPACT PRINTING TECHNOLOGIES. SAN FRANCISCO, AUG. 24 - 28, 1986, SPRINGFIELD, SPSE, US, Bd. CONGRESS 3, 24. August 1986 (1986-08-24), Seiten 16-24, XP000222791

## Beschreibung

Die Erfindung betrifft verbrückte Perinone, Chinophthalone und Perinon-Chinophthalone, Verfahren zu ihrer Herstellung sowie ihre Verwendung zum Massefärben von Kunststoffen.

Es ist bekannt, Perinonfarbstoffe, wie sie beispielsweise aus FR-A-1 075 110 oder US-A-5.466.805 bekannt sind, zum Massefärben von Kunststoffen zu verwenden.

Es ist außerdem bekannt, Chinophthalonfarbstoffe, wie sie beispielsweise in DE-A-44 35 714 oder DE-A-21 32 681 beschrieben sind, zum Massefärben von Kunststoffen einzusetzen.

Derartige Verbindungen zeichnen sich zwar durch eine gute Temperaturstabilität und hohe Lichtechtheit aus, dafür ist allerdings ihre Sublimierechtheit noch verbesserungswürdig. Beim Färbeverfahren, d.h. in den Spritzgussmaschinen, kommt es bei Temperaturen von über 240°C nicht selten zu einer unerwünschten Sublimation des Farbstoffes, wodurch das Arbeitsgerät verschmutzt wird, was aufwendige Reinigungsmaßnahmen erforderlich macht.

Gemäß EP-A-827 986 sind bereits verdoppelte Chinophthalon- und Perinonfarbstoffe mit guter Sublimierechtheit bekannt.

Aufgabe der vorliegenden Erfindung war es, weitere sublimierechte Farbstoffe für das Massefärben von Kunststoffen bereitzustellen, die vorzugsweise auch noch in den monomeren Ausgangsstoffen der Kunststoffe gut löslich sind, um eine größere Anwendungsbreite bei der Einfärbung von Kunststoffen zu gewährleisten.

Es wurden Verbindungen der allgemeinen Formel (I) oder deren tautomeren Formen gefunden 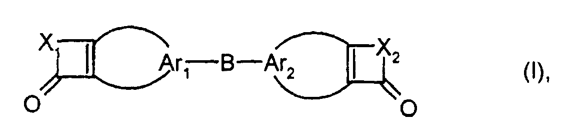 worin
- Ar₁ und Ar₂: unabhängig voneinander Reste zur Vervollständigung gegebenenfalls substituierter carbocyclischer Aromaten bedeuten,
- B: einen Rest der Formel -T₁-W-T₂- bedeutet, worin
T₁ und T₂ unabhängig voneinander für O oder S stehen und
W für Alkylen, insbesondere C₁-C₆-Alkylen, C₆-C₁₀-Arylen, insbesondere Phenylen oder Cycloalkylen steht, die jeweils gegebenenfalls substituiert sind oder für den Rest der Formel (a) 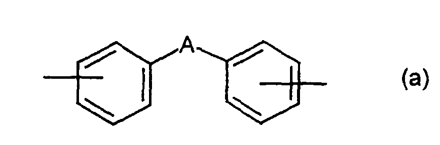 steht, worin die Phenylringe gegebenenfalls substituiert sind und
A für einen Rest der Formel O, S, SO, SO₂, CO, gegebenenfalls substituiertes Alkylen, gegebenenfalls substituiertes Cycloalkylen, wobei das Alkylen oder Cycloalkylen jeweils selbst oder aber über seinen Substituenten mit den benachbarten Phenylringen verbunden sein kann, oder
W für einen Rest der Formeln

-(CH₂)ₛ-O-(CH₂)ₜ-, -(CH₂)ₛ-S-(CH₂)ₜ-,

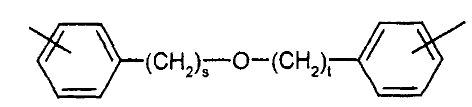

oder 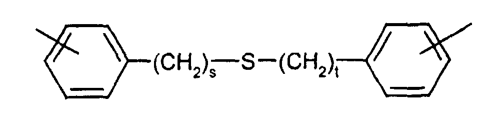 steht, worin
s und t unabhängig voneinander für eine Zahl von 1 bis 6 stehen,
wobei die Enden des zweiwertigen Restes B jeweils an ein aromatisches C-Atom der beiden Reste Ar₁ und Ar₂ gebunden sind,
und
- X₁ und X₂: unabhängig voneinander für einen Rest der Formeln ausgewählt aus der Gruppe 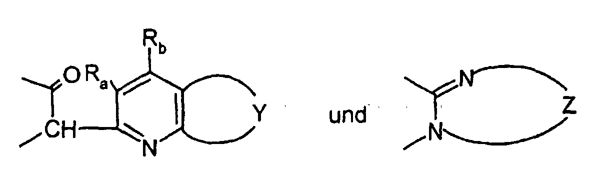 stehen, wobei sie jeweils so im Ring angeordnet sind, dass die 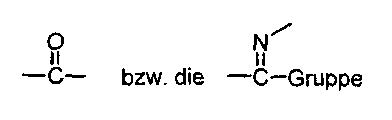 benachbart zur C-C-Doppelbindung steht,
worin
- Y: den Rest eines gegebenenfalls substituierten Benzol- oder Naphthalinringes bildet,
- Z: gegebenenfalls substituiertes ortho-Phenylen, ortho-Naphthylen, peri-(1,8)-Naphthylen oder Arylen aus mehr als zwei miteinander kondensierten Benzolringen bedeutet, wobei die Arylreste, die mehr als zwei miteinander kondensierte Benzolringe aufweisen, in ortho- oder entsprechend einer peri-Stellung im Naphthalin verbrückt sind,
- Rₐ: H oder OH bedeuten und
- R_{b}: H oder Halogen, insbesondere F, Br und Cl bedeutet.

Die peri-Stellung entspricht eigentlich der 1,8-Stellung im Naphthalin. Diese Bedeutung wird allerdings sowohl in der Literatur als auch im Sinne der vorliegenden Anmeldung auch auf Arylene ausgeweitet, die mehr als zwei miteinander kondensierte Benzolringe aufweisen.

Als mögliche Substituenten von Z sowie der beiden Phenylringe des Restes der Formel (a) seien beispielsweise genannt:

C₁-C₆-Alkyl, Halogen, Nitro, Aryl, Aryloxysulfonyl, Hydroxy, C₁-C₆-Alkoxy, Aryloxy, gegebenenfalls durch Alkyl oder Acyl substituiertes Amino, gegebenenfalls durch Alkyl oder Aryl substituiertes Aminosulfonyl, gegebenenfalls durch Alkyl substituiertes Carbonamid oder ein ankondensierter aromatischer, cycloaliphatischer oder heterocyclischer Ring.

Bevorzugte Substituenten sind:

Chlor, Brom, Nitro, Methoxy, NH₂, Benzyloxy, Hydroxy, -SO₂O(C₆H₅), -SO₂N(CH₃)₂, -SO₂NHCH₃, Methyl, Ethyl, n-Propyl, iso-Propyl, n-, sek-, tert.-Butyl, NHCOCH₃, -N(C₂H₅)₂ oder gegebenenfalls substituiertes Phenyl.

Als mögliche Substituenten des durch Y vervollständigten Benzol- bzw. Naphthalinrestes seien beispielsweise genannt: Halogen, insbesondere Cl und Br, -COOH, -COOR, wobei R für C₁-C₁₀-, vorzugsweise C₁-C₄-Alkyl, insbesondere Methyl und Ethyl, C₆-C₁₀-Aryl oder C₅-C₈-Cycloalkyl steht, und C₁-C₆-Alkyl, insbesondere Methyl.

Als mögliche Substituenten des durch Ar₁ und Ar₂ vervollständigten carbocyclischen Aromaten seien beispielsweise genannt: C₁-C₆-Alkyl, insbesondere Methyl, Ethyl, n-Propyl, iso-Propyl, n-, sek- und tert.-Butyl, Halogen, insbesondere Cl und Br, Alkyl- und Arylcarbonyl, Alkyl- und Arylsulfonyl, Nitro, Aryl, insbesondere gegebenenfalls substituiertes Phenyl, Aryloxysulfonyl, insbesondere -SO₂OC₆H₅, Hydroxy, C₁-C₆-Alkoxy, wie Methoxy oder Benzyloxy, Aryloxy, wie Phenoxy gegebenenfalls durch Alkyl oder Acyl substituiertes Amino wie NH₂, NHCOCH₃ und -N(C₂H₅)₂, gegebenenfalls durch Alkyl oder Aryl substituiertes Aminosulfonyl wie SO₂N(CH₃)₂ und SO₂NHCH₃ oder ein ankondensierter aromatischer, cycloaliphatischer oder heterocyclischer Ring.

Besonders bevorzugt bedeuten Ar₁ und Ar₂ unabhängig voneinander einen Rest zur Vervollständigung eines gegebenenfalls substituierten Benzol- oder Naphthalinringes, insbesondere eines gegebenenfalls substituierten Benzolringes.

Als mögliche Substituenten der Alkylengruppe in W und A, die sowohl geradkettig oder verzweigt sein kann, ist beispielsweise Halogen wie F oder Cl, , CF₃, O, S, gegebenenfalls substituiertes Phenyl, C₁-C₆-Alkyl zu nennen.

Als Substituenten der Cycloalkylgruppe, insbesondere der C₅-C₆-Cycloalkylengruppe, besonders bevorzugt der Cyclohexylidengruppe, kommen beispielsweise ein oder mehrere C₁-C₄-Alkylreste in Frage.

Besonders bevorzugt sind Verbindungen der Formel (I), die der Formel (II) oder deren tautomeren Formen entsprechen 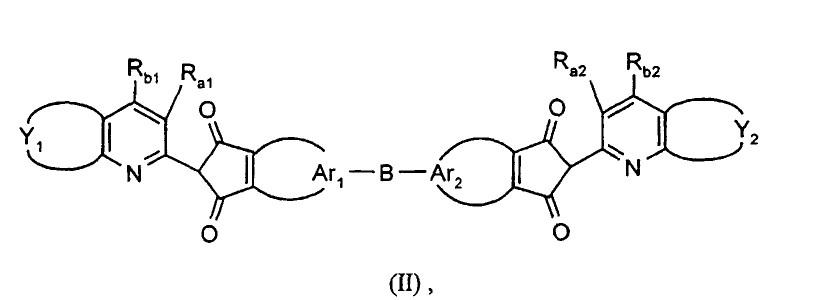 worin
- Y₁ und Y₂: unabhängig voneinander den Rest eines gegebenenfalls substituierten Benzol- oder Naphthalinringes bilden,
- Rₐ₁ und Rₐ₂: unabhängig voneinander H oder OH bedeuten,
- R_{b1} und R_{b2}: unabhängig voneinander H oder Halogen, insbesondere F, Br oder Cl bedeuten und
- Ar₁, Ar₂ und B: die oben angegebene Bedeutung besitzen.

Als mögliche Substituenten für die Reste Y₁ und Y₂ sind beispielsweise die für den Rest Y angegebenen zu nennen.

Besonders bevorzugt bedeuten R_{b1} und R_{b2} jeweils Wasserstoff.

Besonders bevorzugt sind Verbindungen der Formel (II), die der Formel (IIa) oder deren tautomeren Formen entsprechen 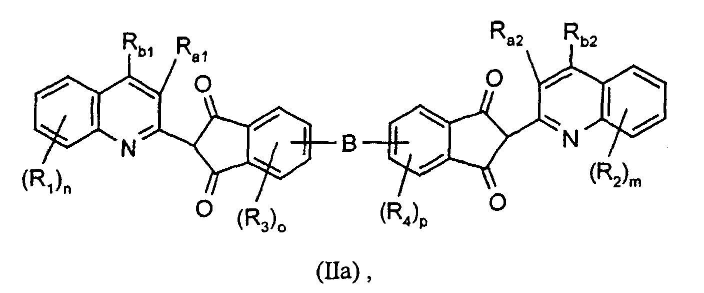 worin
- n und m: unabhängig voneinander für eine Zahl von 0 bis 4 stehen,
- R₁ und R₂: unabhängig voneinander jeweils gleich oder verschiedene Bedeutung besitzen, wie sie für die Substituenten des durch Y₁ und Y₂ vervollständigten Reste angegeben sind,
- o und p: unabhängig eine Zahl von 0 bis 2, insbesondere 0 oder 1 bedeuten,
- R₃ und R₄: unabhängig voneinander jeweils gleich oder verschiedene Bedeutung annehmen können, wie sie für die Substituenten der durch Ar₁ bzw. Ar₂ vervollständigten carbocyclischen Aromaten angegeben sind,
- R_{b1} und R_{b2}: die obengenannte Bedeutung haben, vorzugsweise für H stehen,
- Rₐ₁ und Rₐ₂: die obengenannte Bedeutung haben, vorzugsweise für OH stehen und
- B: die obengenannte Bedeutung hat.

Als bevorzugte Brückenglieder B sind zu nennen

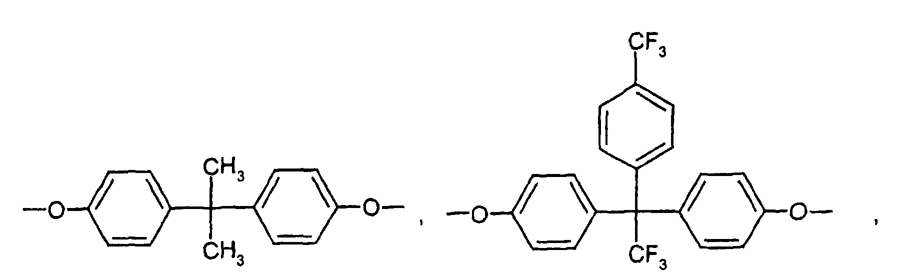

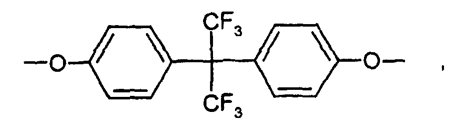

-O-CH₂-CH₂-O-,

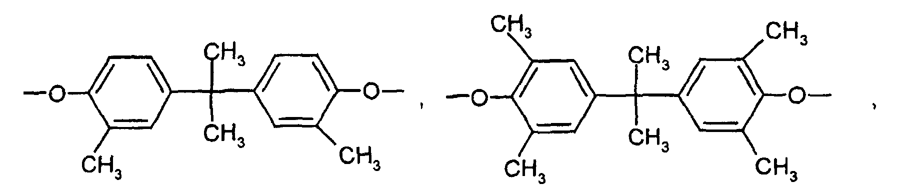

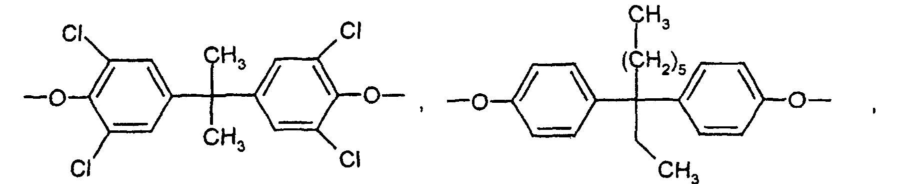

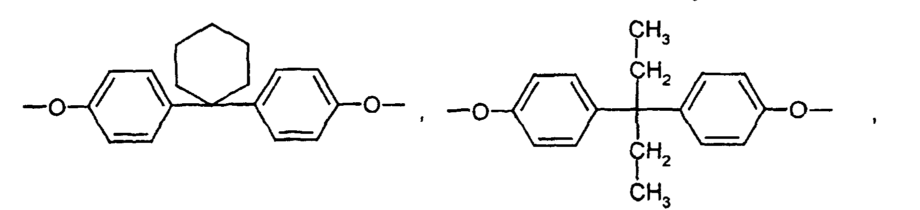

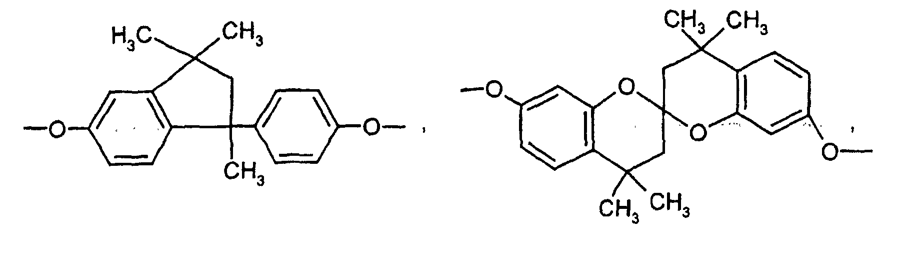

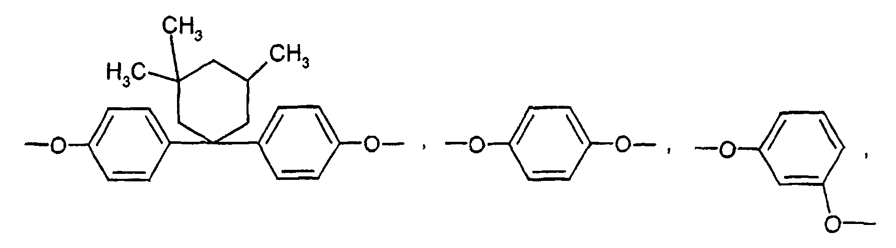

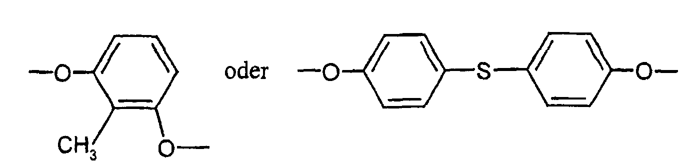

sowie die entsprechenden Dithio-Verbindungen (T₁ und T₂ = S).

Ebenfalls bevorzugte Reste B sind solche, worin W oder A folgende Bedeutung hat:
-CH₂-, -CH₂-CH₂-, -CH₂CH₂CH₂-, -CH₂CH₂CH₂CH₂-,

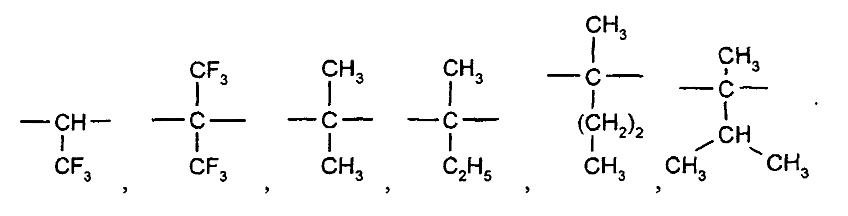

Ganz besonders bevorzugt sind Verbindungen der Formel (II), worin
Y₁ = Y₂
Rₐ₁ = Rₐ₂
R_{b1} = R_{b2} und
Ar₁ = Ar₂ bedeuten.

Entsprechend vorteilhaft sind Verbindungen der Formel (IIa), worin
n = m,
R₁ = R₂,
Rₐ₁ = Rₐ₂,
R_{b1} = R_{b2}
o = p und
R₃ = R₄ bedeutet,
wobei insbesondere
n, m, o und p für 0 stehen.

Besonders bevorzugt sind Verbindungen der Formel (II), die der Formel (IIb) oder deren tautomere Formen entsprechen 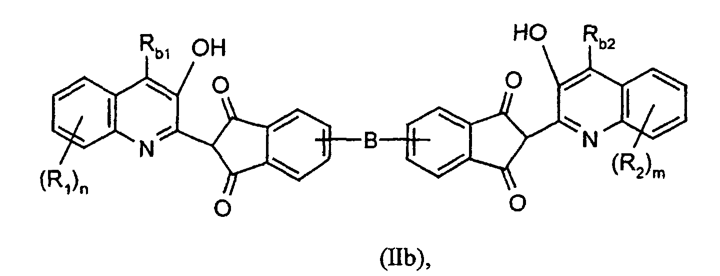 worin
n, m, R₁, R₂, R_{b1}, R_{b2} und B die oben angegebenen Bedeutungen haben.

Die erfindungsgemäßen Verbindungen der Formel (I) zeichnen sich durch eine hervorragende Sublimierechtheit beim Massefärben von Kunststoffen aus. Zudem besitzen sie eine sehr gute Lichtechtheit, gute Thermostabilität und eine besonders hohe Farbstärke. Ein Vergleich in der Farbstärke beispielsweise der Verbindungen der Formel (II) gegenüber den nicht verdoppelten Chinophthalonen zeigt eine Zunahme, die größer als erwartet ist (bezogen auf die Molekulargewichtszunahme).

Besitzen die erfindungsgemäßen Chinophthalone der Formel (II) unterschiedliche Bedeutungen für Y₁ und Y₂ und/oder Rₐ₁ und Rₐ₂, und/oder Ar₁ und Ar₂, so kann es vorteilhaft sein, die gegebenenfalls herstellungsbedingt entstehenden Mischungen von symmetrischen und unsymmetrischen Chinophthalonen dem Verwendungszweck zuzuführen.

Ebenfalls bevorzugt sind Verbindungen der Formel (I), die der Formel (III) oder deren tautomeren Formen entsprechen 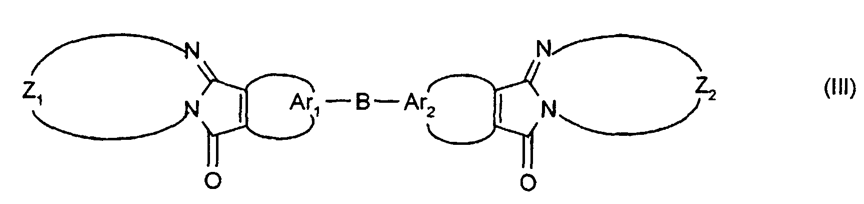
- Z₁ und Z₂: unabhängig voneinander gegebenenfalls substituiertes ortho-Phenylen, ortho-Naphthylen, peri-(1,8)-Naphthylen oder Arylen aus mehr als zwei miteinander kondensierten Benzolringen bedeuten, wobei die Arylreste, die mehr als zwei miteinander kondensierte Benzolringe aufweisen, in orthooder entsprechend einer peri-Stellung im Naphthalin verbrückt sind,
und
- Ar₁, Ar₂ und B: die oben angegebenen Bedeutungen besitzen.

Als mögliche Substituenten für die Reste Z₁ und Z₂ sind beispielsweise die für den Rest Z angegebenen zu nennen.

Besonders bevorzugte Reste Z₁ und Z₂ entsprechen unabhängig voneinander einem gegebenenfalls substituierten peri-(1,8)-Naphthylen.

Besonders bevorzugt sind Verbindungen der Formel (III), die der Formel (IIIa) entsprechen 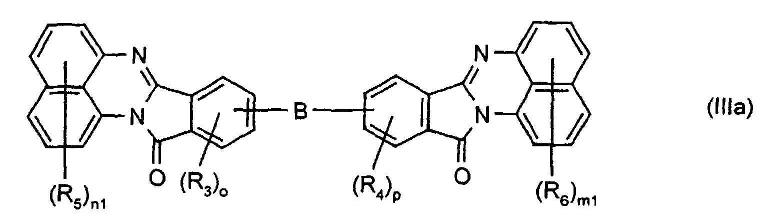 worin
- n₁ und m₁: unabhängig voneinander für eine Zahl von 0 bis 4 stehen,
- R₅ und R₆: unabhängig voneinander jeweils gleich oder verschieden sind und die Bedeutung der für die Reste Z₁ und Z₂ genannten Substituenten annehmen können, insbesondere für Halogen und C₁-C₆-Alkyl, stehen,
- o und p: unabhängig voneinander eine Zahl von 0 bis 2, insbesondere 0 oder 1, bedeuten,
- R₃ und R₄: unabhängig voneinander jeweils gleich oder verschieden sind und die Bedeutung der für die Reste Z₁ und Z₂ genannten Substituenten annehmen können, insbesondere Halogen, NO₂, -NH-Acyl oder -NH-Alkyl bedeuten und
- B: die obengenannte Bedeutung hat.

Die bevorzugte Bedeutung von B entspricht der oben angegebenen.

Ganz besonders bevorzugt sind Verbindungen der Formel (III),
worin
Z₁ = Z₂ und
Ar₁ = Ar₂ bedeuten.

Entsprechend vorteilhaft sind Verbindungen der Formel (IIIa),
worin
n₁ = m₁,
R₅ = R₆,
o = p und
R₃ = R₄ bedeuten,
wobei insbesondere
n₁, m₁, o und p für 0 stehen.

Besonders bevorzugt sind Verbindungen der Formel (III), die der Formel (IIIb) oder (IIIc) entsprechen 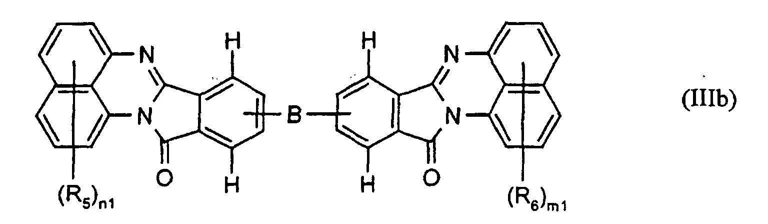 oder 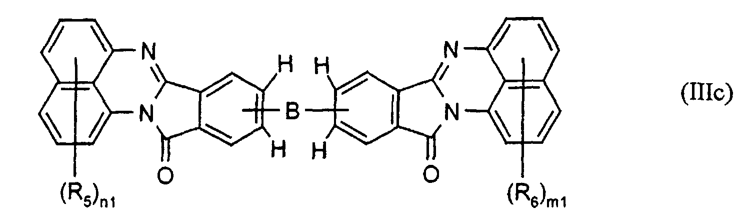 worin
n₁, m₁, R₅, R₆ und B die oben angegebenen Bedeutungen haben.

Die erfindungsgemäßen Verbindungen der Formel (III) zeichnen sich ebenfalls durch eine hervorragende Sublimierechtheit beim Massefärben von Kunststoffen aus. Zudem besitzen sie eine sehr gute Lichtechtheit, sehr gute Thermostabilität und eine besonders hohe Farbstärke.

Besitzen die erfindungsgemäßen Perinone der Formel (III) unterschiedliche Bedeutungen für Z₁ und Z₂ und/oder für Ar₁ und Ar₂, so kann es vorteilhaft sein, die gegebenenfalls herstellungsbedingt entstehenden Mischungen von symmetrischen und unsymmetrischen Perinonen der Formel (III) dem Verwendungszweck zuzuführen.

Ebenfalls bevorzugt sind Verbindungen der Formel (I), die der Formel (IV) oder deren tautomeren Formen entsprechen 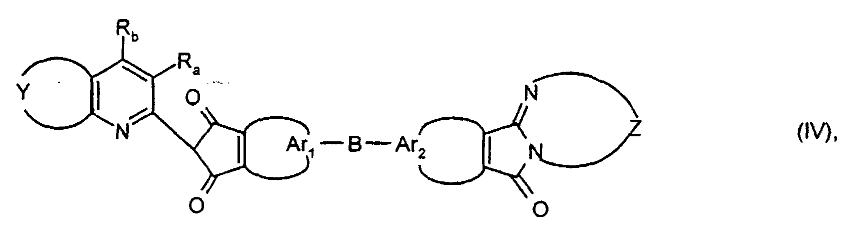 worin
Y, Z, Rₐ, R_{b}, Ar₁, Ar₂ und B die oben angegebene Bedeutung besitzen.

Besonders bevorzugt sind Verbindungen der Formel (IV), die der Formel (IVa) oder deren tautomeren Formen entsprechen 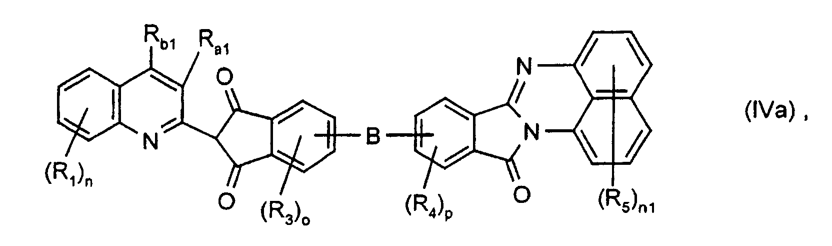 worin
R₁, R₃, R₄, R₅, R_{b1}, Rₐ₁, B, n, p und n₁ die oben angegebene Bedeutungen besitzen.

Ganz besonders bevorzugt sind Verbindungen der Formel (IV), die der Formel (IVb) oder deren tautomeren Formen entsprechen 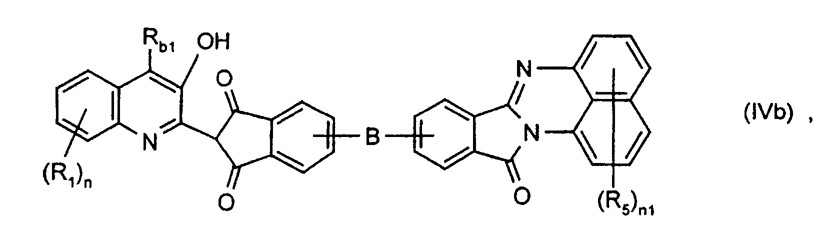 worin
R₁, R₅, R_{b1}, B, n und n₁ die oben angegebene Bedeutung besitzen.

Die erfindungsgemäßen Verbindungen der Formel (IV) zeichnen sich ebenfalls durch eine hervorragende Sublimierechtheit beim Massefärben von Kunststoffen aus. Zudem besitzen sie eine sehr gute Lichtechtheit, gute Thermostabilität und eine besonders hohe Farbstärke.

Die Thermostabilität ist bei 300°C und deutlich darüber sehr gut.

Besonders bevorzugt sind herstellungsbedingte Mischungen enthaltend Verbindungen der Formel (IV) sowie solche der Formeln (II) und (III). Dabei sind insbesondere solche Mischungen von Vorteil, welche neben der Verbindung der Formel (IV) 0 bis 25 Gew.-% einer Verbindung der Formel (II) und 0 bis 25 Gew.-% einer Verbindung der Formel (III) enthalten, wobei die Summe aus den Verbindungen (IV), (II) und (III) 100 % beträgt.

Verbindungen der Formel (V) sind beispielsweise beschrieben in Plaste und Kautschuk, 28. Jahrgang, Heft 11/1981, S. 601-606.

Die Erfindung betrifft weiterhin ein Verfahren zur Herstellung der erfindungsgemäßen Verbindungen der Formel (I), das dadurch gekennzeichnet ist, dass man Tetracarbonsäuren oder deren Anhydride der Formel (V) 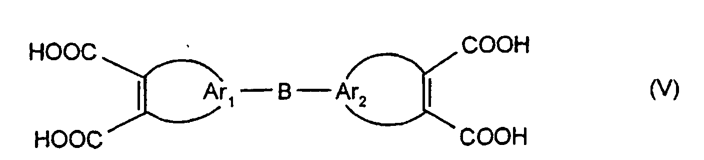 worin
Ar₁, Ar₂ und B die obengenannte Bedeutung besitzen,
mit einer oder mehreren Verbindungen der Formeln (VI) und/oder (VII) 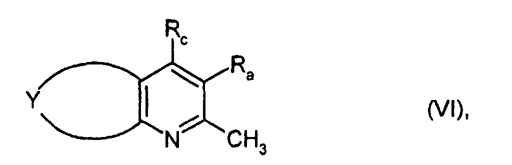 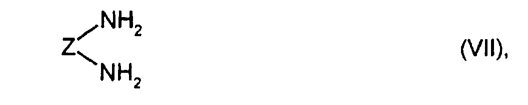 worin
- R_{c}: H, COOH oder Halogen, insbesondere F, Br und Cl bedeutet, insbesondere für H oder COOH steht und
- Y, Z und Rₐ: die oben angegebene Bedeutung besitzen,
kondensiert, wobei die Summe der Verbindungen der Formeln (VI) und (VII) zwei Mol-Äquivalenten, bezogen auf die Tetracarbonsäure (V) entspricht.

Die Angabe der Mol-Äquivalente der zur Herstellung der Verbindung (I) eingesetzten Reaktanden dient lediglich zur Angabe der Stöchiometrie und schließt nicht größere oder kleinere Mengen aus, die gegebenenfalls technisch sinnvoller sind.

Die eingesetzten Verbindungen der Formeln (VI) bzw. (VII) können in ihrer Summe selbstverständlich auch mehr als 2 Mol-Äquivalente, bezogen auf die Tetracarbonsäure (V) bzw. deren Anhydride, betragen. Bevorzugt ist es jedoch, stöchiometrisch zu arbeiten.

Die Kondensation kann dabei direkt durch Zusammenschmelzen äquimolarer Mengen der Komponenten der Formeln (V) und (VI) bzw. (VII) bei einer Temperatur von 120°C bis 250°C erfolgen, oder vorteilhafter durch Umsetzung in einem Lösungsmittel bei einer Temperatur von 110°C bis 220°C, gegebenenfalls unter Druck, durchgeführt werden, wobei eine destillative Entfernung des Reaktionswassers erfolgen kann.

Geeignete Lösungsmittel sind beispielsweise: Chlorbenzol, o-Dichlorbenzol, Trichlorbenzol, Xylol, Dimethylformamid, N-Methylpyrrolidon, Eisessig, Propionsäure, Phenol, Kresole, Phenoxyethanol, Glykole und deren Mono- und Dialkylether, Alkohole, z.B. Methanol, Ethanol, i-Propanol, n-Butanol und Wasser.

Gegebenenfalls kann die Reaktion unter Zusatz eines sauren Katalysators wie beispielsweise: Zinkchlorid, p-Toluolsulfonsäure, Salzsäure, Schwefelsäure, organische Säuren u.a. erfolgen.

Besonders bevorzugt wird die Tetracarbonsäure der Formel (V) in Form ihres Anhydrids eingesetzt.

Bevorzugt werden in das erfindungsgemäße Verfahren Verbindungen der Formel (V) eingesetzt, die der Formel (Va) oder deren Anhydriden entsprechen 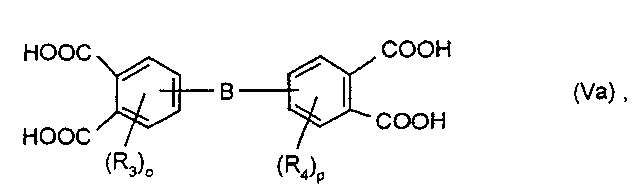 worin
R₃, R₄, B, o und p die obengenannten Bedeutungen besitzen.

Ganz besonders bevorzugt werden Verbindungen der Formel (V) eingesetzt, die der Formel (Vb), Formel (Vc) oder deren jeweiligen Anhydriden entsprechen 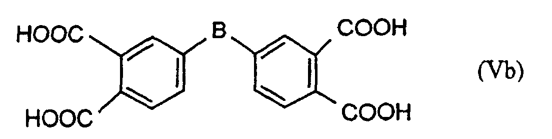 oder 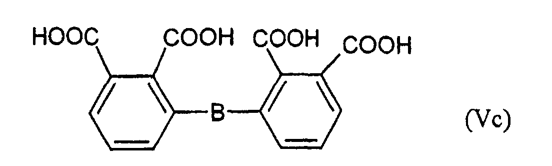 wobei
- B: die oben genannte bevorzugte Bedeutung besitzen

Bevorzugte Verbindungen der Formel (VI) sind Chinaldine der Formel (VIa) 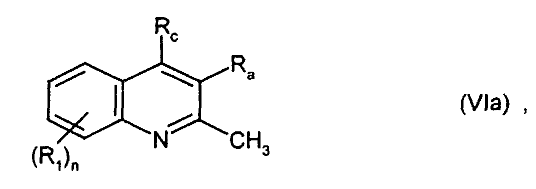 worin
R₁, R_{c}, Rₐ und n die oben angegebene Bedeutung haben.

Geeignete Chinaldine der Formel (VIa) sind beispielsweise solche der nachfolgend genannten Formeln:

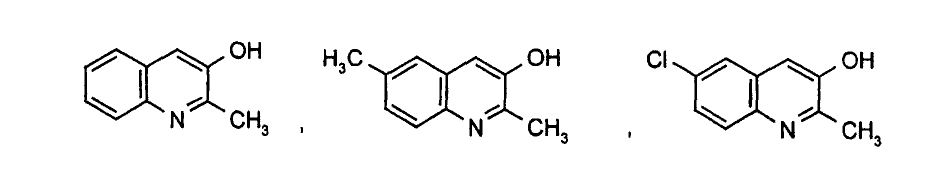

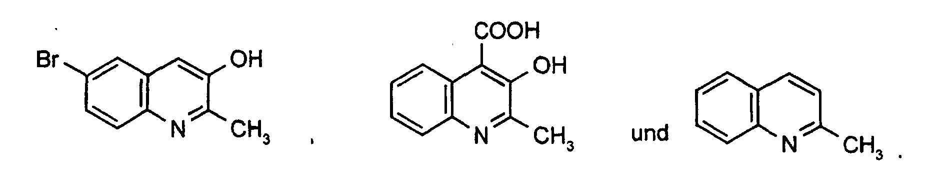

Die Diamine der Formel (VII) sind bekannt oder lassen sich beispielsweise analog bekannten Diaminen herstellen.

Als aromatische Diamine der Formel (VII) sind bevorzugt:
o-Phenylendiamin, Chlor-o-phenylendiamine, Dichlor-o-phenylendiamine, Methyl-ophenylendiamine, Ethyl-o-phenylendiamine, Methoxy-o-phenylendiamine, Acetamino-o-phenylendiamine, Phenyl-o-phenylendiamine, Naphthylen-o-diamine, ferner 1,8-Naphthylendiamin, Chlor-1,8-naphthylendiamine, Dichlor-1,8-naphthylendiamine, Methyl-1,8-naphthylendiamine, Dimethyl-1,8-naphthylendiamine, Methoxy-1,8-naphthylendiamine, Ethoxy-1,8-naphthylendiamine, Acetamino-1,8-naphthylendiamine und 1,8-Diaminoacetnaphthylen.

In einer weiteren bevorzugten Verfahrensvariante wird ein gegebenenfalls substituiertes peri-Naphthylendiamin als Verbindung der Formel (VII) eingesetzt, insbesondere 1,8-Naphthylendiamin.

Bevorzugt ist das erfindungsgemäße Verfahren zur Herstellung von Verbindungen der Formel (I), die der Formel (II) entsprechen, das dadurch gekennzeichnet ist, dass man Tetracarbonsäuren oder deren Anhydride der Formel (V) mit Verbindungen der Formeln (VIb) und/oder (VIc) 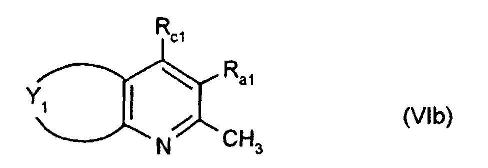 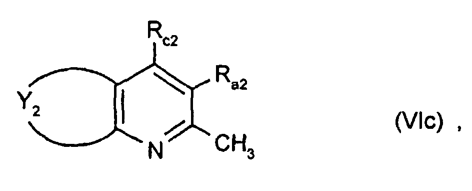 worin
- R_{c1} und R_{c2}: unabhängig voneinander H, COOH oder Halogen, insbesondere F, Br und Cl bedeuten, insbesondere für H oder COOH stehen und
- Rₐ₁, Rₐ₂, Y₁ und Y₂: die obengenannte Bedeutung besitzen,
kondensiert, wobei die Summe der Verbindungen (VIb) und (VIc) zwei Mol-Äquivalenten, bezogen auf die Tetracarbonsäure (V) entspricht.

Die Kondensation kann dabei direkt durch Zusammenschmelzen äquimolarer Mengen der Komponenten der Formeln (V) und (VIb) bzw. (VIc) bei einer Temperatur von 160°C bis 250°C, vorzugsweise 180 bis 220°C, besonders bevorzugt 190 bis 200°C erfolgen, oder vorteilhafter durch Umsetzung in einem Lösungsmittel bei einer Temperatur von 110°C bis 220°C, vorzugsweise 160 bis 180°C, gegebenenfalls unter Druck durchgeführt werden, wobei eine destillative Entfernung des Reaktionswassers erfolgen kann.

Bevorzugte Verbindungen der Formel (VIb) bzw. (VIc) sind Chinaldine der Formel (VIbb) 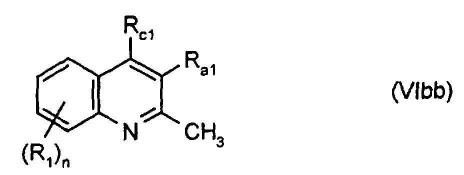 bzw. der Formel (VIcc) 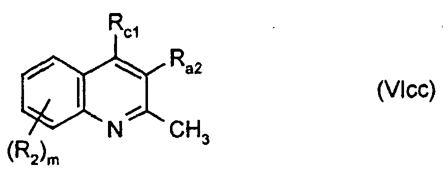 worin
R₁, R₂, R_{c1}, R_{c2}, Rₐ₁, Rₐ₂, n und m die oben angegebene Bedeutung haben.

Die Aufarbeitung eines Reaktionsansatzes zur Herstellung der Verbindungen der Formel (II) erfolgt vorzugsweise durch Verdünnen mit Alkoholen wie Methanol, Ethanol, Propanol oder Butanol. Auch aromatische Verdünnungsmittel wie Chlorbenzol oder Toluol sowie Ligroin können verwendet werden. Dieses erfindungsgemäße Verfahren liefert die erfindungsgemäße Verbindung vorzugsweise in Ausbeuten von 85 bis 95 % der Theorie.

Gegebenenfalls kann sich an die Kondensation eine Halogenierung anschließen, insbesondere Chlorierung oder Bromierung, wobei diese unter an sich bekannten Bedingungen erfolgt. So führt beispielsweise die Bromierung von Verbindungen der Formel (II) mit R_{b1} = R_{b2} = H in Eisessig bei einer Temperatur von 80 bis 120°C zu Verbindungen der Formel (II), worin R_{b1} und R_{b2} Brom bedeuten.

Das erfindungsgemäße Verfahren eignet sich ebenfalls bevorzugt zur Herstellung der Verbindungen der Formel (I), die der Formel (III) entsprechen, das dadurch gekennzeichnet ist, dass man Tetracarbonsäuren oder deren Anhydride der Formel (V) mit Verbindungen der Formeln (VIIa) und/oder (VIIb) kondensiert 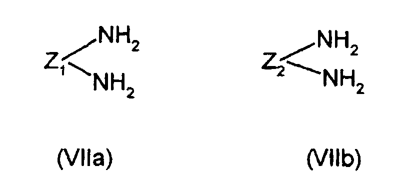 wobei die Summe der eingesetzten Diamine (VIIa) und (VIIb) zwei Mol-Äquivalente, bezogen auf die Tetracarbonsäure (V) entspricht und Z₁ und Z₂ die obengenannte Bedeutung besitzen.

Die Kondensation zur Herstellung von Verbindungen der Formel (III) kann dabei direkt durch Zusammenschmelzen äquimolarer Mengen der Komponenten der Formeln (V) und (VIIa) bzw. (VIIb) bei einer Temperatur von 120°C bis 250°C, vorzugsweise bei 120 bis 180°C, erfolgen, oder vorteilhafter durch Umsetzung in einem Lösungsmittel bei einer Temperatur von 80°C bis 220°C, vorzugsweise bei 120 bis 180°C, gegebenenfalls unter Druck, durchgeführt werden, wobei eine destillative Entfernung des Reaktionswassers erfolgen kann.

Verbindungen der Formel (III), die gegebenenfalls Substituenten aus der Gruppe der Alkyl-, Arylaminosulfonylreste tragen, lassen sich beispielsweise aus den entsprechenden Verbindungen der Formel (III), in denen ein Substituent einen Chlorsulfonylrest bedeutet, mit Alkyl- bzw. Arylaminen darstellen.

Erfindungsgemäße Verbindungen der Formel (III), in denen ein Substituent ein Aryloxysulfonylrest ist, können auch durch Umsetzung der entsprechenden Chlorsulfonyl-Farbstoffe mit Phenolen oder Naphtholen in Gegenwart einer Base, z.B. Pyridin, Triethylamin, Alkali- bzw. Erdalkalicarbonaten, -hydroxiden oder -oxiden gewonnen werden.

Verbindungen der Formel (III), in denen Substituenten für Alkyloxy oder Acyloxy stehen, können zusätzlich durch Alkylierung bzw. Acylierung der erfindungsgemäßen Verbindungen, die eine Hydroxygruppe tragen, dargestellt werden.

Jene Verbindungen der Formel (III) mit einer gegebenenfalls acylierten bzw. alkylierten Aminogruppe können außerdem durch Reduktion mit üblichen Reduktionsmitteln, z.B. Eisen, Zink, Natriumsulfid, Wasserstoff u.a. der entsprechenden Verbindungen, in denen der entsprechende Substituent für eine Nitrogruppe steht, und gegebenenfalls nachfolgende Acylierung bzw. Alkylierung gewonnen werden. Der Acylierungsschritt kann auch im Zuge der Reduktion durch Zusatz eines Acylierungsmittels erfolgen.

Die Aufarbeitung des Reaktionsansatzes bei der Herstellung von Verbindungen der Formel (III) erfolgt vorzugsweise durch Verdünnen mit Alkoholen wie Methanol, Ethanol, Propanol oder Butanol. Auch aromatische Verdünnungsmittel wie Chlorbenzol oder Toluol sowie Ligroin können verwendet werden. Das erfindungsgemäße Verfahren liefert die erfindungsgemäßen Verbindungen, vorzugsweise in Ausbeuten von 90 bis 95 % der Theorie.

Das erfindungsgemäße Verfahren ist ebenfalls bevorzugt zur Herstellung von Verbindungen der Formel (I), die der Formel (IV) entsprechen, das dadurch gekennzeichnet ist, dass man Tetracarbonsäuren oder deren Anhydride der Formel (V) mit Verbindungen der Formel (VI) und Diaminen der Formel (VII) kondensiert, wobei die Substituenten der genannten Verbindungen die oben angegebenen Bedeutungen haben und die Summe der Verbindungen der Formeln (VI) und (VII) zwei Mol-Äquivalente, bezogen auf die Tetracarbonsäure (V), beträgt. Als bevorzugte Verbindungen der Formeln (V) bis (VII) kommen die oben angegebenen in Frage.

Das Verhältnis der Verbindungen (VI) und (VII) zueinander kann in weiten Bereichen variieren. Es beträgt beispielsweise (VI) zu (VII) = 10:90 bis 90:10. Bevorzugt ist das Verhältnis etwa 1:1.

Die Kondensation kann dabei direkt durch Zusammenschmelzen äquimolarer Mengen der Komponenten der Formeln (V) und (VI) und (VII) bei einer Temperatur von 160°C bis 250°C, vorzugsweise 180 bis 220°C, besonders bevorzugt 190 bis 200°C erfolgen, oder vorteilhafter in einem Lösungsmittel bei einer Temperatur von 110°C bis 220°C, vorzugsweise 160 bis 180°C, gegebenenfalls unter Druck durchgeführt werden, wobei eine destillative Entfernung des Reaktionswassers erfolgen kann.

Die erfindungsgemäßen Verbindungen der Formel (I) eignen sich hervorragend zum Färben von Kunststoffen in der Masse.

Unter Massefärben werden hierbei insbesondere Verfahren verstanden, bei denen der Farbstoff in die geschmolzene Kunststoffmasse eingearbeitet wird, z.B. unter Zuhilfenahme eines Extruders, oder bei denen der Farbstoff bereits Ausgangskomponenten zur Herstellung des Kunststoffes, z.B. Monomeren vor der Polymerisation, zugesetzt wird.

Besonders bevorzugte Kunststoffe sind Thermoplaste, beispielsweise Vinylpolymere, Polyester und Polyamide.

Geeignete Vinylpolymere sind Polystyrol, Styrol-Acrylnitril-Copolymere, Styrol-Butadien-Copolymere, Styrol-Butadien-Acrylnitril-Terpolymere, Polymethacrylat, Polyvinylchlorid u.a.

Weiterhin geeignete Polyester sind: Polyethylenterephthalate, Polycarbonate und Celluloseester.

Bevorzugt sind Polystyrol, Styrol-Mischpolymere, Polycarbonate und Polymethacrylat. Besonders bevorzugt ist Polystyrol.

Die erwähnten hochmolekularen Verbindungen können einzeln oder in Gemischen, als plastische Massen oder Schmelzen vorliegen.

Die erfindungsgemäßen Farbstoffe werden in feinverteilter Form zur Anwendung gebracht, wobei Dispergiermittel mitverwendet werden können aber nicht müssen.

Werden die Verbindungen (I) nach der Polymerisation eingesetzt, so werden sie mit dem Kunststoffgranulat trocken vermischt oder vermahlen und dieses Gemisch z.B. auf Mischwalzen oder in Schnecken plastifiziert und homogenisiert. Man kann die Farbstoffe aber auch der schmelzflüssigen Masse zugeben und diese durch Rühren homogen verteilen. Das derart vorgefärbte Material wird dann wie üblich z.B. durch Verspinnen zu Borsten, Fäden usw. oder durch Extrusion oder im Spritzguss-Verfahren zu Formteilen weiterverarbeitet.

Da die Farbstoffe der Formel (I) gegenüber Polymerisationskatalysatoren, insbesondere Peroxiden, beständig sind, ist es auch möglich, die Farbstoffe den monomeren Ausgangsmaterialien für die Kunststoffe zuzusetzen und dann in Gegenwart von Polymerisationskatalysatoren zu polymerisieren. Dazu werden die Farbstoffe vorzugsweise in den monomeren Komponenten gelöst oder mit ihnen innig vermischt.

Die erfindungsgemäßen Farbstoffe der Formel (I) sind besonders gut löslich in monomeren Ausgangsmaterialien für Kunststoffe (z.B. Methylmethacrylat).

Die Farbstoffe der Formel (I) werden vorzugsweise zum Färben der genannten Polymeren in Mengen von 0,0001 bis 1 Gew.-%, insbesondere 0,01 bis 0,5 Gew.-%, bezogen auf die Polymermenge, eingesetzt.

Durch Zusatz von in den Polymeren unlöslichen Pigmenten, wie z.B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-%, bezogen auf die Polymermenge, verwendet werden.

Nach dem erfindungsgemäßen Verfahren können auch Mischungen verschiedener Farbstoffe der Formel (I) und/oder Mischungen von Farbstoffen der Formel (I) mit anderen Farbstoffen und/oder anorganischen bzw. organischen Pigmenten eingesetzt werden.

Die Verbindungen der Formel (II) sind gelb, die der Formel (III) und die der Formel (IV) orange.

Die Erfindung wird erläutert, jedoch nicht beschränkt auf die folgenden Beispiele, in denen die Teile gewichtsmäßig angegeben sind. Prozentangaben bedeuten Gewichtsprozente (Gew.-%).

### Beispiel 1

30 g N-Methyl-2-pyrrolidin (NMP) wurden unter Rühren mit 5,2 g (0,01 mol) eines Bisphthalsäureanhydrides der Formel 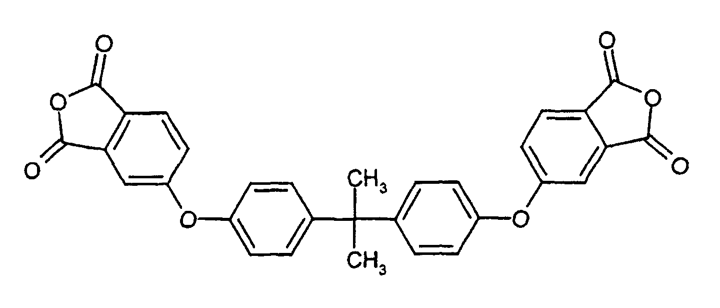 und 3,2 g (0,02 mol) 1,8-Naphthalindiamin der Formel 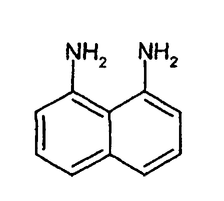 versetzt und auf 120°C erwärmt. Es wurde 4 h lang bei Reaktionstemperatur gerührt. Danach ließ man auf Raumtemperatur abkühlen und versetzte die Reaktionsmischung langsam über ca. 1 Stunde mit 150 ml Methanol. Der kristalline Niederschlag wurde abgesaugt und mehrfach mit Methanol gewaschen. Anschließend wurde mit heißem Wasser gewaschen und bei 70°C im Vakuum getrocknet.

Ausbeute: 6,8 g (89%)

Der Farbstoff besitzt die Formel

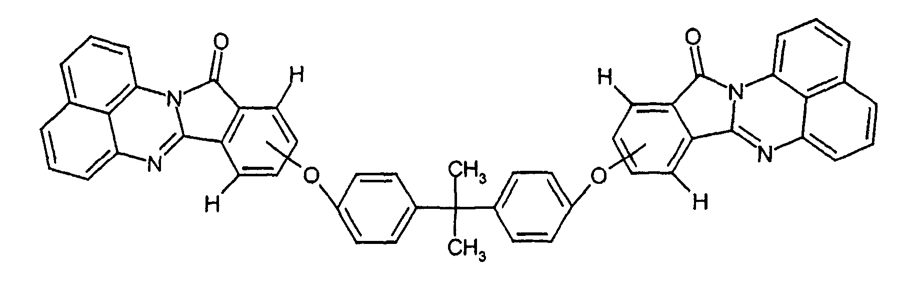

Der Farbstoff färbt Kunststoffe wie Polyamid 6, ABS, Polyester und Polystyrol in klaren neutralen Orangetönen von sehr guten Echtheiten und ist bei den notwendigen Verabeitungstemperaturen in den Kunststoffschmelzen sehr gut löslich.

Anstelle von N-Methylpyrrolidon (NMP) wurde als Lösungsmittel für die Synthese auch Phenol, o-Dichlorbenzol, Nitrobenzol und Ditolylether eingesetzt, wobei gleiche Ergebnisse erzielt wurden.

Im Vergleich zum entsprechenden unverbrückten Farbstoff der Formel 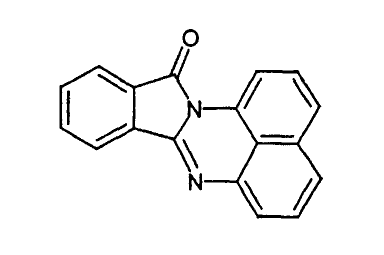 zeigt sich eine deutlich verbesserte Sublimierechtheit. Während der unverbrückte Farbstoff bereits bei 350°C deutlich und bei weiterem Erhitzen auf 450°C vollständig sublimiert, zeigt der verbrückte Farbstoff selbst bei dieser Temperatur eine hervorragende Sublimierechtheit.

Der Farbstoff ist bei Raumtemperatur bereits sehr gut in Methylmethacrylat löslich. Im Gegensatz dazu ist der in Beispiel 16 von EP-A-827 986 erwähnte verbrückte Farbstoff der Formel 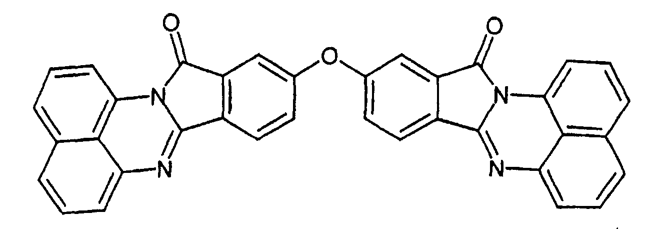 in MMA praktisch völlig unlöslich. Ähnliches gilt für organische Lösungsmittel, wie N-Methyl-2-pyrrolidon, Dimethylformamid, Dichlormethan und Aceton. Dies ist vorteilhaft für die Reinigung von Maschinen, die zur Kunststoffeinfärbung benutzt werden.

In Kunststoffschmelzen von PA 6, ABS, Polystyrol, Polypropylen und Polyethylen ist der Farbstoff deutlich besser zu verarbeiten als der in EP-A-827 986, Beispiel 16 beschriebene Farbstoff.

Es wurden durch analoges Vorgehen aus den entsprechenden Bisphthalsäureanhydriden folgende Perinonfarbstoffe hergestellt, die die gleichen vorteilhaften Eigenschaften aufweisen.

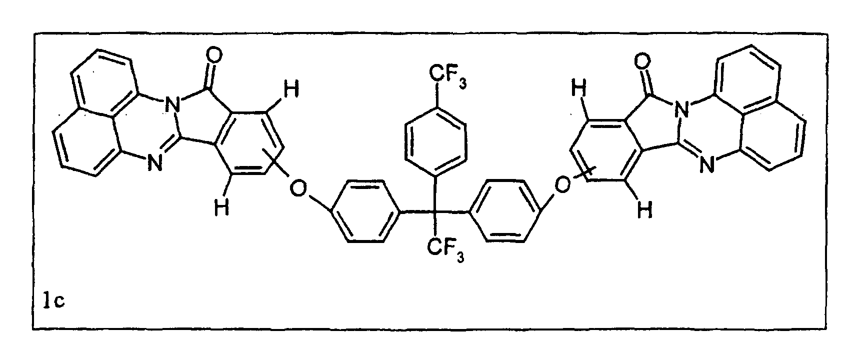

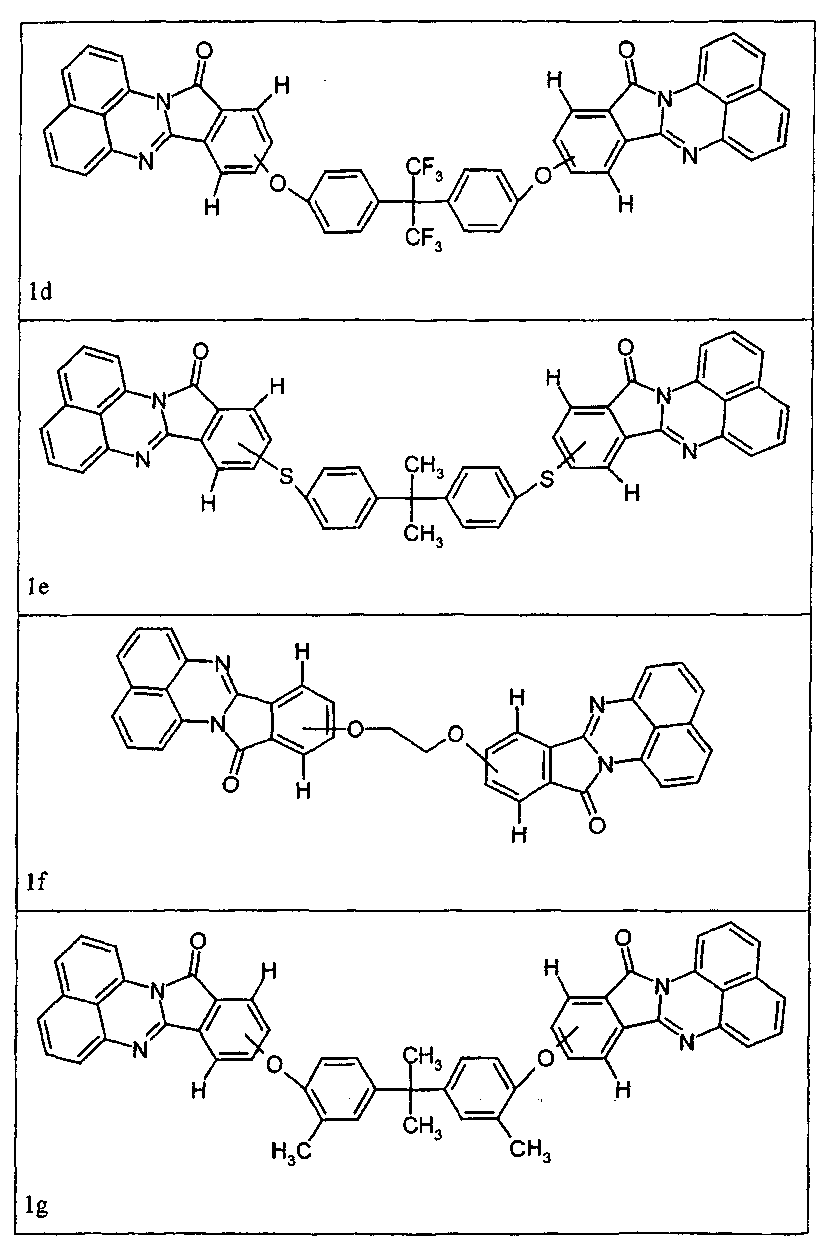

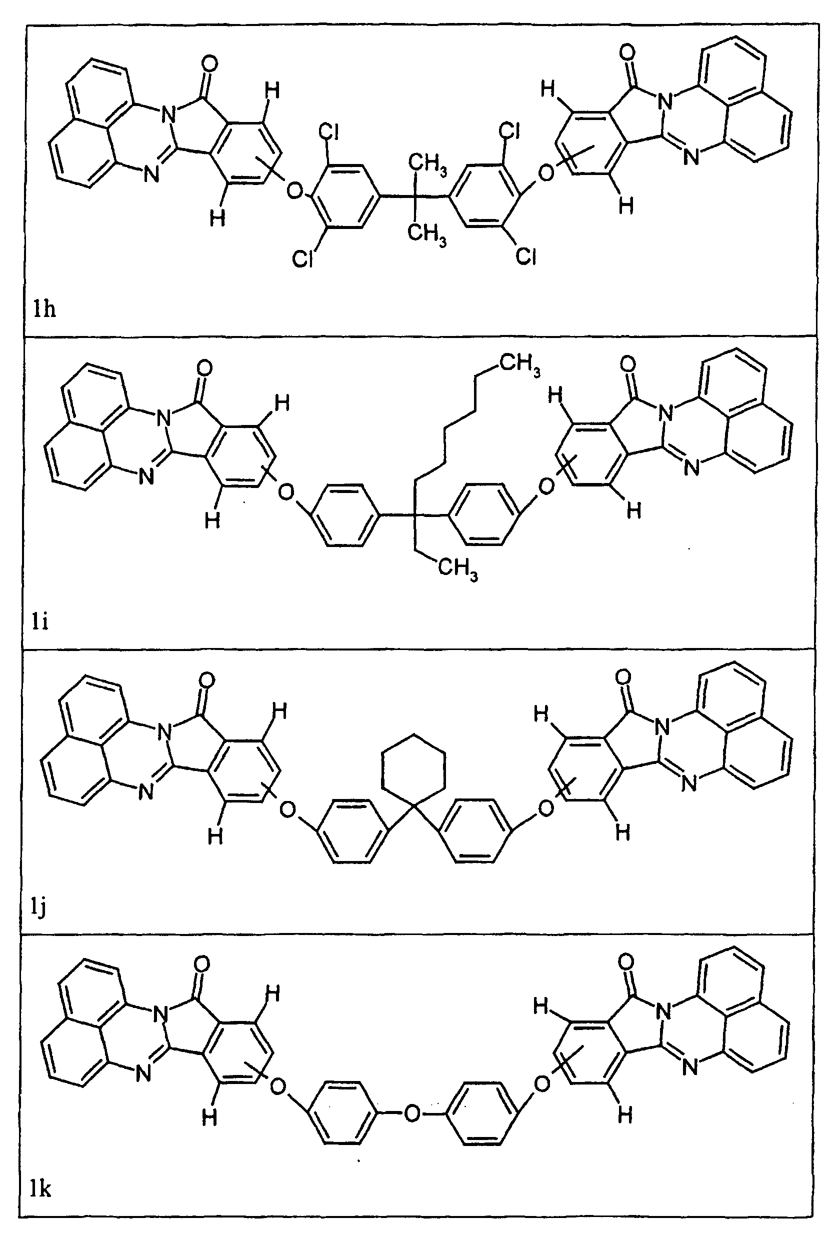

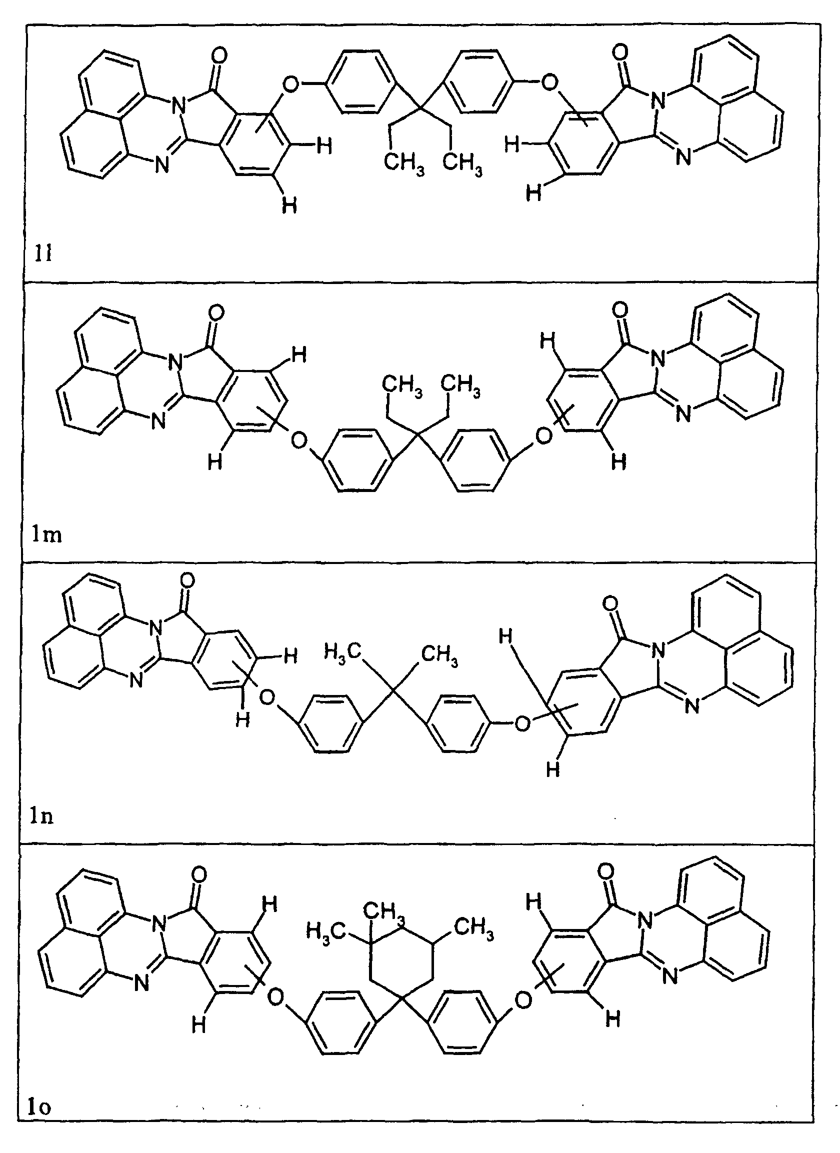

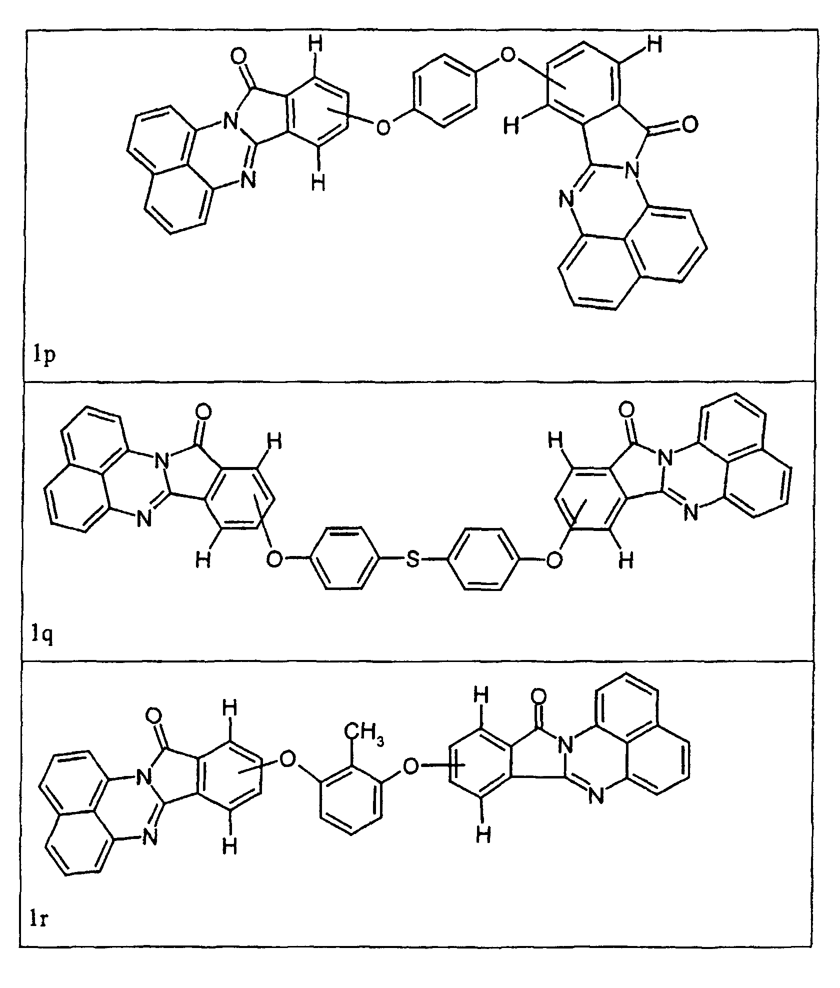

### Beispiel 2

50 g Phenol wurden bei 70°C unter Rühren mit 5,2 g (0,010 mol) eines Bisphthalsäureanhydrides der Formel 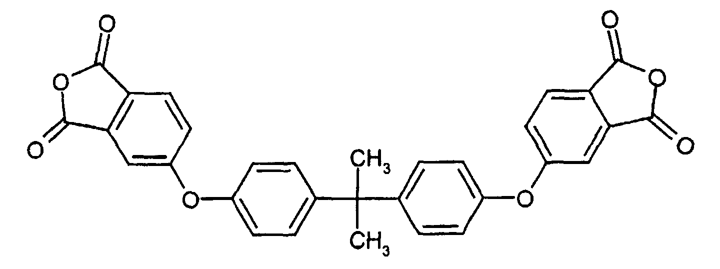 und 4,1 g (0,02 mol) 3-Hydroxychinaldin-4-carbonsäure der Formel 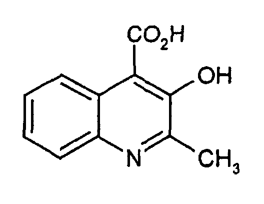 versetzt und auf 175°C erwärmt. Es wurde 12 h lang bei Reaktionstemperatur gerührt, wobei Reaktionswasser abdestillierte und Kohlendioxid aus der 3-Hydroxychinaldin-4-carbonsäure abgespalten wurde. Danach ließ man auf 80°C abkühlen und versetzte die Reaktionsmischung langsam über ca. 1 Stunde mit 150 ml Methanol, wobei die Temperatur zu Beginn auf 65 bis 70°C sank und danach in diesem Bereich gehalten wurde. Der kristalline Niederschlag wurde abgesaugt und mehrfach mit Methanol gewaschen. Anschließend wurde mit heißem Wasser gewaschen und bei 70°C im Vakuum getrocknet.

Ausbeute: 7,2 g (90 %)

Der Farbstoff besitzt die Formel

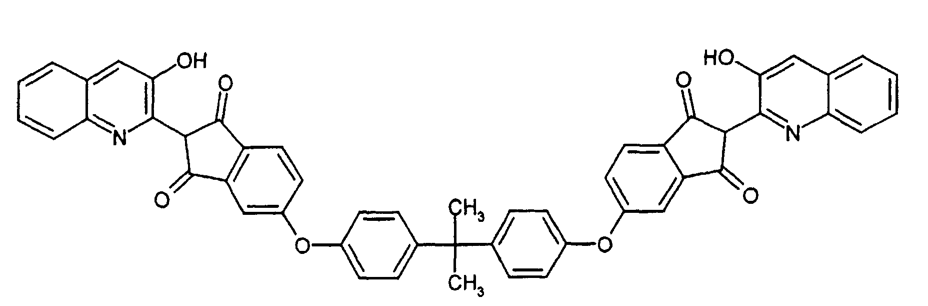

Anstelle von Phenol wurde als Lösungsmittel auch o-Dichlorbenzol, Nitrobenzol, N-Methyl-2-pyrrolidon (NMP) und Ditolylether eingesetzt, wobei gleiche Ergebnisse erzielt wurden.

Der Farbstoff färbt Kunststoffe wie ABS, Polyester und Polystyrol in klaren neutralen Gelbtönen von sehr guten Echtheiten.

Es wurden durch analoges Vorgehen aus den entsprechenden Bisphthalsäureanhydriden folgende Chinophthalonfarbstoffe hergestellt.

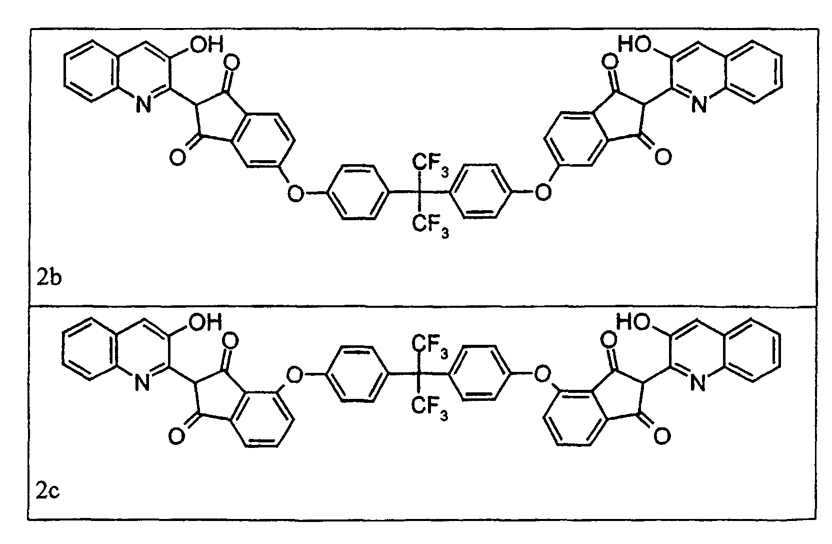

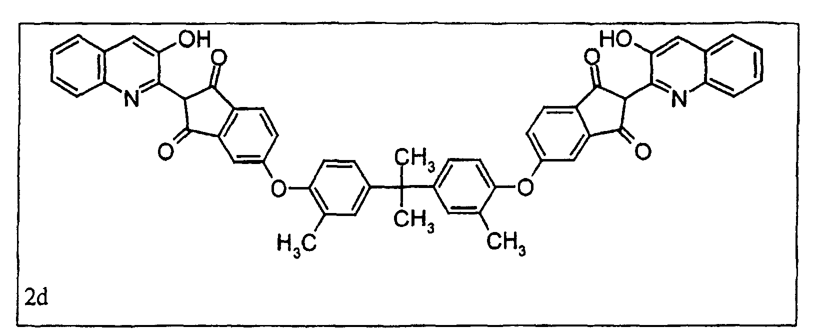

Alle Farbstoffe ergeben bei der Einfärbung von Kunststoffen wie ABS, Polystyrol und Polyester klare gelbe Farbtöne mit sehr guten Echtheiten.

### Beispiel 3

47 g Phenol wurden bei 70°C unter Rühren mit 5,2 g (0,010 mol) eines Bisphthalsäureanhydrides der Formel 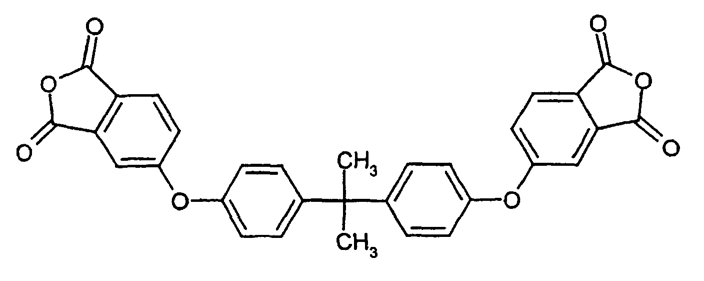 und 2,0 g (0,01 mol) 3-Hydroxychinaldin-4-carbonsäure sowie 1,6 g (0,01 mol) 1,8-Naphthalindiamin versetzt und auf 175°C erwärmt. Es wurde 12 h lang bei Reaktionstemperatur gerührt, wobei Reaktionswasser abdestillierte und Kohlendioxid aus der 3-Hydroxychinaldin-4-carbonsäure abgespalten wurde. Danach ließ man auf 80°C abkühlen und versetzte die Reaktionsmischung langsam über ca. 1 Stunde mit 150 ml Methanol, wobei die Temperatur zu Beginn auf 65 bis 70°C sank und danach in diesem Bereich gehalten wurde. Der kristalline Niederschlag wurde abgesaugt und mehrfach mit Methanol gewaschen. Anschließend wurde mit heißem Wasser gewaschen und bei 70°C im Vakuum getrocknet.

Ausbeute: 7,0 g (90 %)

Der Farbstoff besitzt die Formel 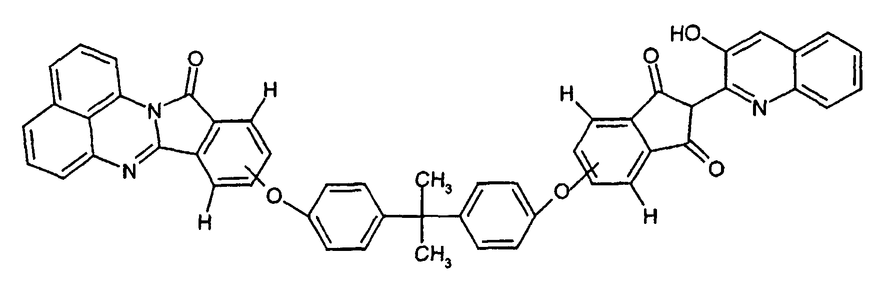 und enthält jeweils ca. 10 % des beidseitigen (symmetrischen) Chinophthalons bzw. Perinons.

Anstelle von Phenol wurde als Lösungsmittel auch o-Dichlorbenzol, Nitrobenzol, N-Methyl-2-pyrrolidon (NMP) und Ditolylether eingesetzt, wobei gleiche Ergebnisse erzielt wurden.

Der Farbstoff färbt Kunststoffe wie ABS, Polyester und Polystyrol in klaren Orangetönen von sehr guten Echtheiten.

Es wurden durch analoges Vorgehen aus den entsprechenden Bisphthalsäureanhydriden, Diaminen und Chinaldinen folgende Farbstoffe hergestellt.

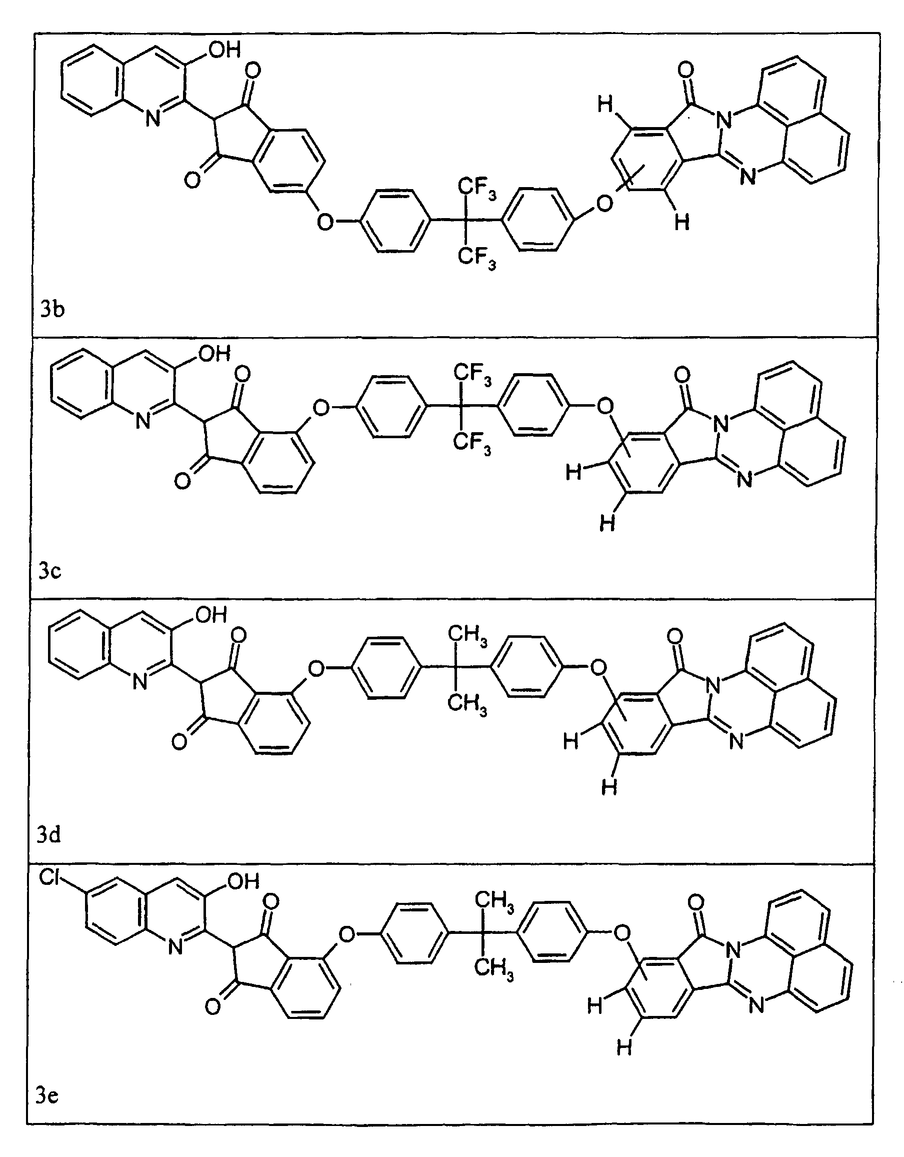

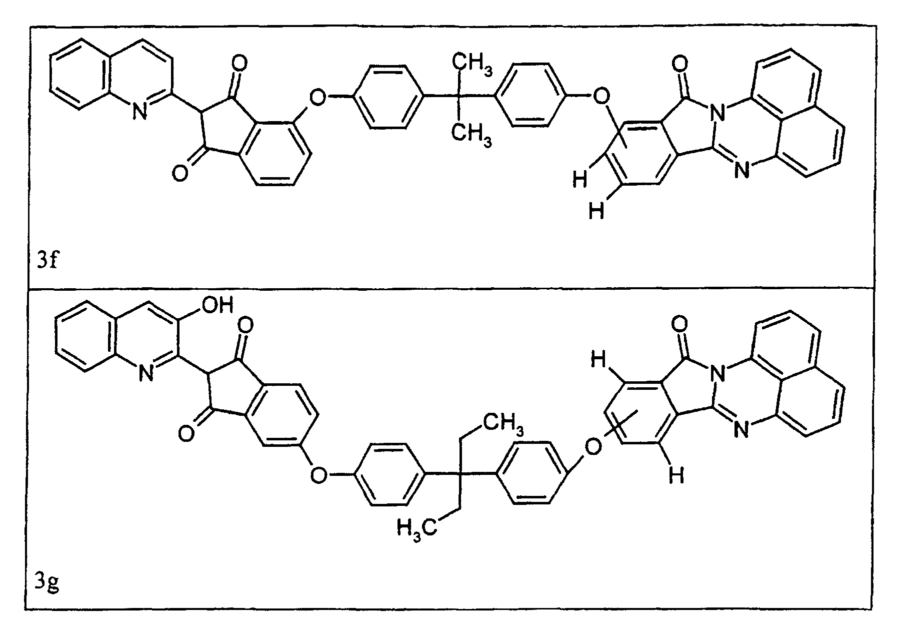

Alle Farbstoffe ergeben bei der Einfärbung von Kunststoffen wie ABS, Polystyrol und Polyester klare Orangetöne mit sehr guten Echtheiten.

### Beispiel 4

47 g (0,5 mol) Phenol wurden bei 70°C unter Rühren mit 5,2 g (0,010 mol) eines Bisphthalsäureanhydrides der Formel 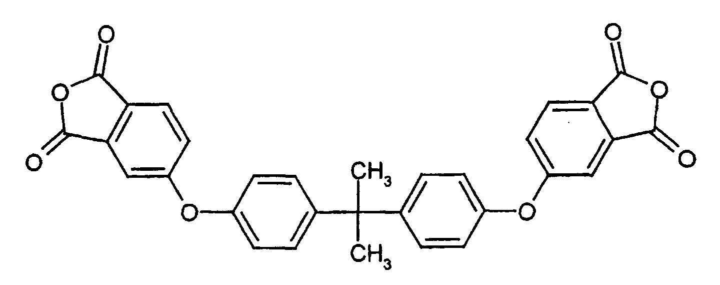 und 2,9 g (0,20 mol) Chinaldin der Formel 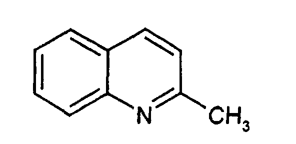 versetzt und auf 175°C erwärmt. Es wurde 12 h lang bei Reaktionstemperatur gerührt, wobei Reaktionswasser abdestillierte. Danach ließ man auf 80°C abkühlen und versetzte die Reaktionsmischung langsam über ca. 1 Stunde mit 150 ml Methanol, wobei die Temperatur zu Beginn auf 65 bis 70°C sank und danach in diesem Bereich gehalten wurde. Der kristalline Niederschlag wurde abgesaugt und mehrfach mit Methanol gewaschen. Anschließend wurde mit heißem Wasser gewaschen und bei 70°C im Vakuum getrocknet.

Ausbeute: 7,2 g (93 %)

Der Farbstoff besitzt die Formel 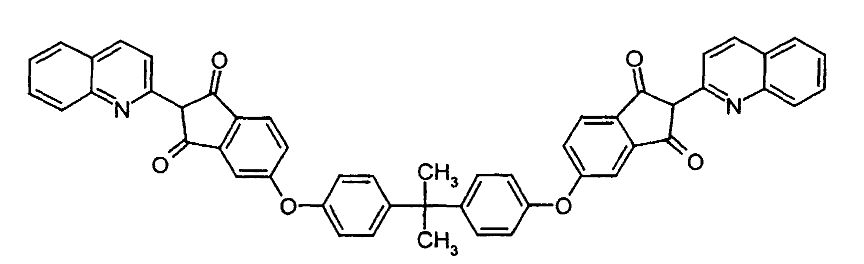 Anstelle von Phenol wurde als Lösungsmittel auch o-Dichlorbenzol, Nitrobenzol, N-Methyl-2-pyrrolidon (NMP) und Ditolylether eingesetzt, wobei gleiche Ergebnisse erzielt wurden.

Der Farbstoff färbt Kunststoffe wie ABS, Polyester und Polystyrol in klaren grünstichigen Gelbtönen von sehr guten Echtheiten.

Es wurden durch analoges Vorgehen aus den entsprechenden Bisphthalsäureanhydriden und den entsprechenden Chinaldinderivaten folgende Chinophthalonfarbstoffe hergestellt.

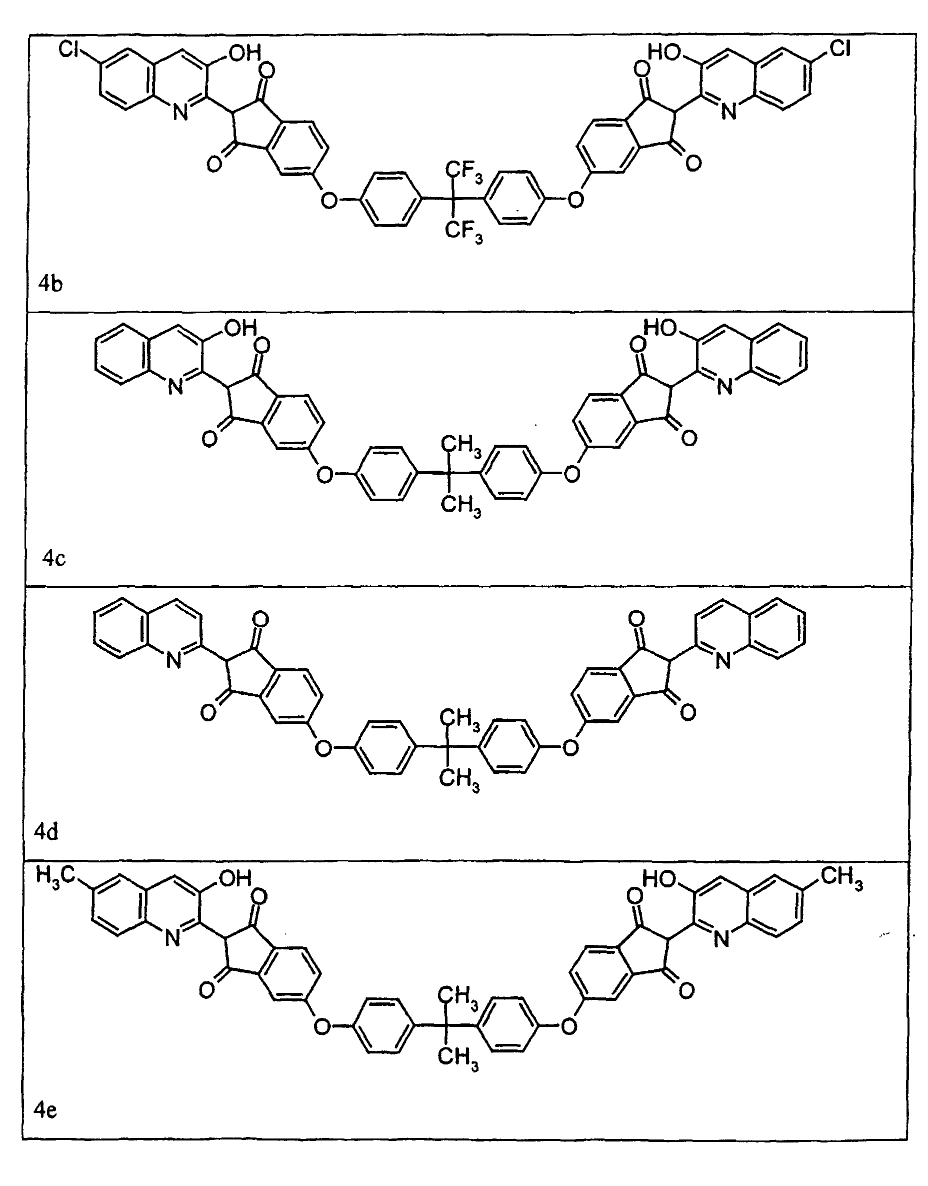

### Beispiel 5

50 g (0,5 mol) Phenol wurden bei 70 °C unter Rühren mit 5,2 g (0,010 mol) eines Bisphthalsäureanhydrides der Formel 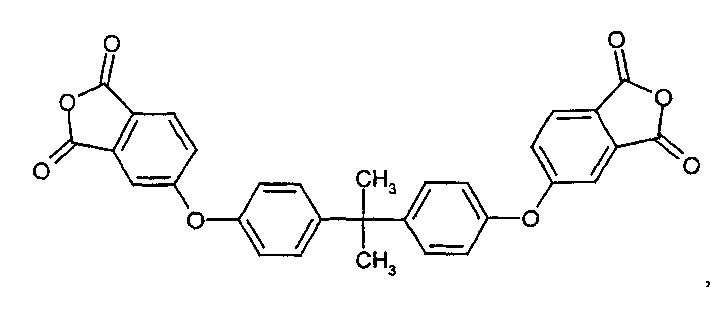 1,4 g (0,010 mol) Chinaldin der Formel 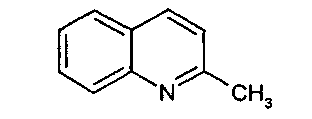 und 1,6 g (0,10 mol) Chinaldin der Formel 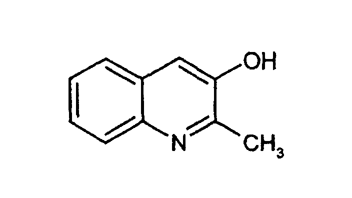 versetzt und auf 175°C erwärmt. Es wurde 12 h lang bei Reaktionstemperatur gerührt, wobei Reaktionswasser abdestillierte und Kohlendioxid aus der 3-Hydroxychinaldin-4-carbonsäure abgespalten wurde. Danach ließ man auf 80°C abkühlen und versetzte die Reaktionsmischung langsam über ca. 1 Stunde mit 150 ml Methanol, wobei die Temperatur zu Beginn auf 65 bis 70°C sank und danach in diesem Bereich gehalten wurde. Der kristalline Niederschlag wurde abgesaugt und mehrfach mit Methanol gewaschen. Anschließend wurde mit heißem Wasser gewaschen und bei 70°C im Vakuum getrocknet.

Ausbeute: 7,2 g (90 %)

Der Farbstoff besitzt die Formel

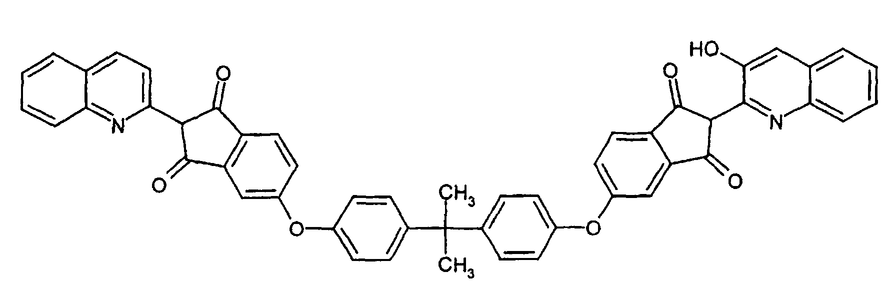

Daneben wurden auch jeweils ca. 10 % der beiden möglichen symmetrischen Farbstoffe erhalten.

Anstelle von Phenol wurde als Lösungsmittel auch o-Dichlorbenzol, Nitrobenzol, N-Methyl-2-pyrrolidon (NMP) und Ditolylether eingesetzt, wobei gleiche Ergebnisse erzielt wurden.

Der Farbstoff färbt Kunststoffe wie ABS, Polyester und Polystyrol in klaren Gelbtönen von sehr guten Echtheiten.

Es wurden durch analoges Vorgehen aus den entsprechenden Bisphthalsäureanhydriden und den entsprechenden Chinaldinderivaten folgende Chinophthalonfarbstoffe hergestellt.

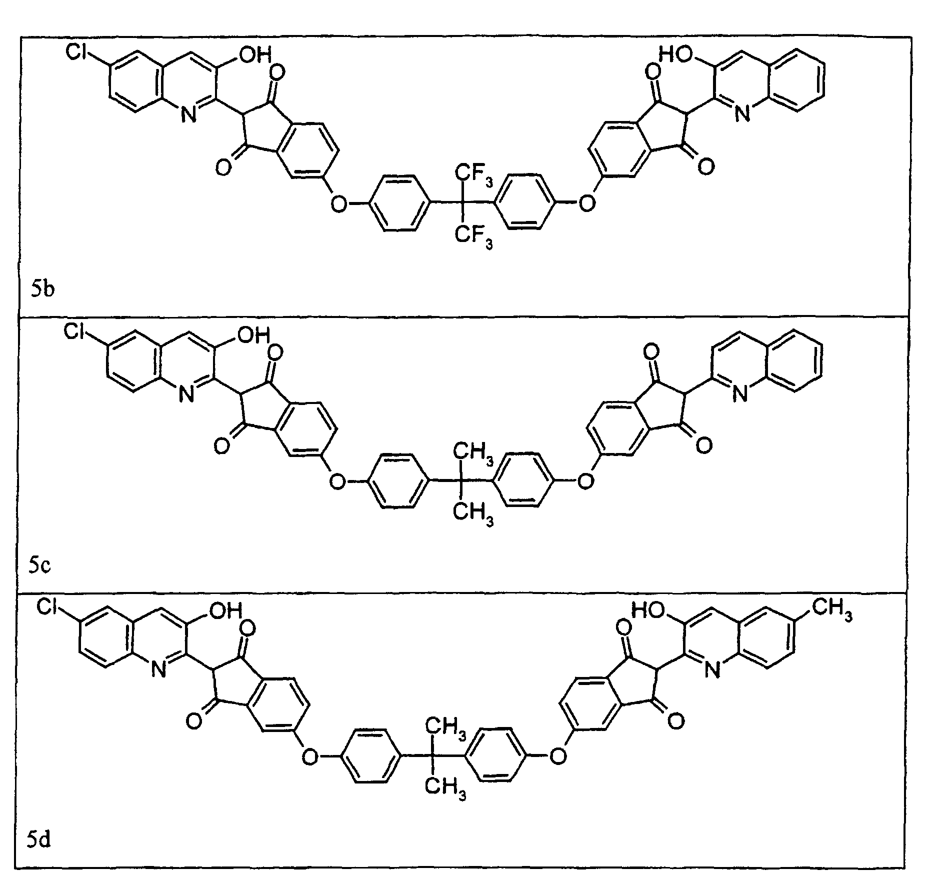

### Beispiel 6

30 g N-Methyl-2-pyrrolidon (NMP) wurden unter Rühren mit 5,2 g (0,010 mol) eines Bisphthalsäureanhydrides der Formel 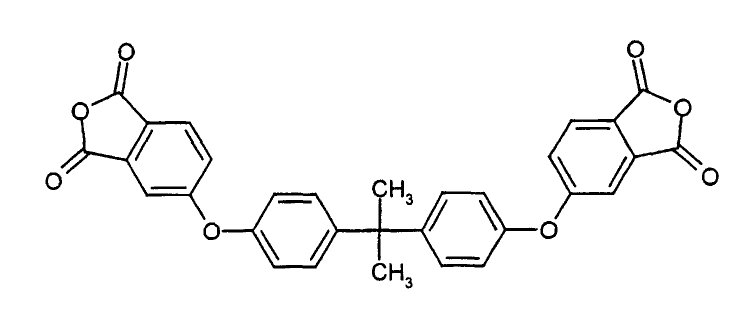 und 1,60 g (0,01 mol) 1,8-Naphthalindiamin der Formel 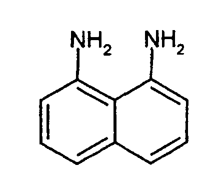 und 1,10 g (0,01 mol) o-Phenylendiamin der Formel 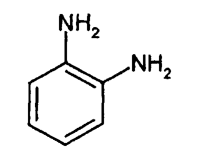 versetzt und auf 120°C erwärmt. Es wurde 4 h lang bei Reaktionstemperatur gerührt. Danach ließ man auf Raumtemperatur abkühlen und versetzte die Reaktionsmischung langsam über ca. 1 Stunde mit 150 ml Methanol. Der kristalline Niederschlag wurde abgesaugt und mehrfach mit Methanol gewaschen. Anschließend wurde mit heißem Wasser gewaschen und bei 70°C im Vakuum getrocknet.

Ausbeute: 6,4 g (89 %)

Der Farbstoff besitzt die Formel

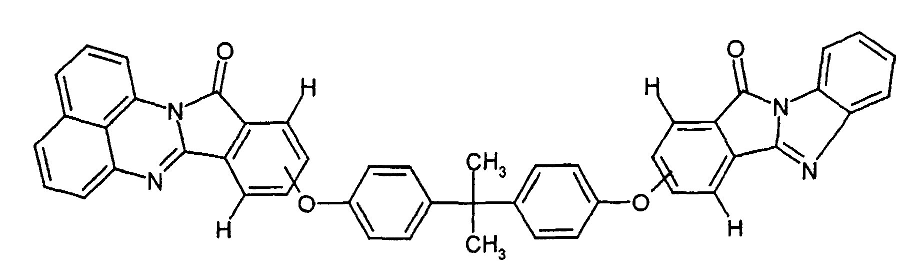

Der Farbstoff färbt Kunststoffe wie Polyamid 6, ABS, Polyester und Polystyrol in klaren Orangetönen von sehr guten Echtheiten und ist bei den notwendigen Verabeitungstemperaturen in den Kunststoffschmelzen sehr gut löslich.

Anstelle von NMP wurde als Lösungsmittel für die Synthese auch Phenol, o-Dichlorbenzol, Nitrobenzol und Ditolylether eingesetzt, wobei gleiche Ergebnisse erzielt wurden.

## Patentansprüche

1. Verbindungen der allgemeinen Formel (I) oder deren tautomeren Formen 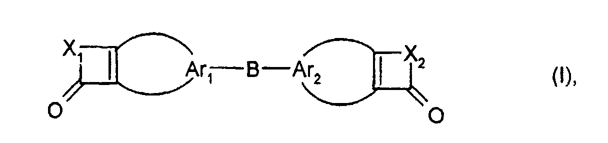 worin
Ar₁ und Ar₂ unabhängig voneinander Reste zur Vervollständigung gegebenenfalls substituierter carbocyclischer Aromaten bedeuten,
B einen Rest der Formel -T₁-W-T₂- bedeutet, worin
T₁ und T₂ unabhängig voneinander für O oder S stehen und
W für Alkylen, insbesondere C₁-C₆-Alkylen, C₆-C₁₀-Arylen, insbesondere Phenylen oder Cycloalkylen steht, die jeweils gegebenenfalls substituiert sind oder für den Rest der Formel (a) 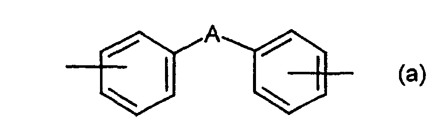 steht, worin die Phenylringe gegebenenfalls substituiert sind und
A für einen Rest der Formel O, S, SO, SO₂, CO, gegebenenfalls substituiertes Alkylen, gegebenenfalls substituiertes Cycloalkylen, wobei das Alkylen oder Cycloalkylen jeweils selbst oder aber über seinen Substituenten mit den benachbarten Phenylringen verbunden sein kann, oder
W für einen Rest der Formeln
-(CH₂)ₛ-O-(CH₂)ₜ-, -(CH₂)ₛ-S-(CH₂)ₜ- . 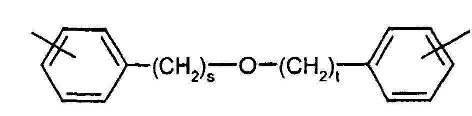
oder 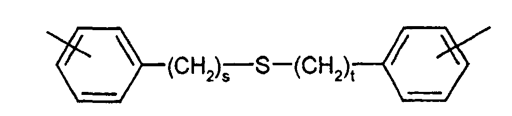 steht, worin
s und t unabhängig voneinander für eine Zahl von 1 bis 6 stehen,
wobei die Enden des zweiwertigen Restes B jeweils an ein aromatisches C-Atom der beiden Reste Ar₁ und Ar₂ gebunden sind,
und
X₁ und X₂ unabhängig voneinander für einen Rest der Formeln ausgewählt aus der Gruppe 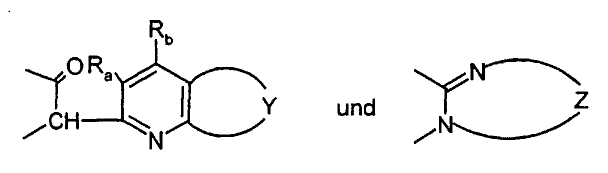
stehen, wobei sie jeweils so im Ring angeordnet sind, dass die 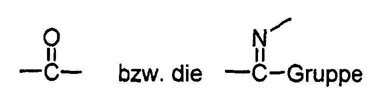 benachbart zur C-C-Doppelbindung steht,
worin
Y den Rest eines gegebenenfalls substituierten Benzol- oder Naphthalinringes bildet,
Z gegebenenfalls substituiertes ortho-Phenylen, ortho-Naphthylen, peri-(1,8)-Naphthylen oder Arylen aus mehr als zwei miteinander kondensierten Benzolringen bedeutet, wobei die Arylreste, die mehr als zwei miteinander kondensierte Benzolringe aufweisen, in orthooder entsprechend einer peri-Stellung im Naphthalin verbrückt sind,
Rₐ H oder OH bedeuten und
R_{b} H oder Halogen, insbesondere F, Br und Cl bedeutet.

2. Verbindungen gemäß Anspruch 1, **dadurch gekennzeichnet, dass** Ar₁ und Ar₂ unabhängig voneinander einen Rest zur Vervollständigung eines gegebenenfalls substituierten Benzol- oder Naphthalinringes, insbesondere eines gegebenenfalls substituierten Benzolringes bedeuten.

3. Verbindungen gemäß Anspruch 1, die der Formel (II) oder deren tautomeren Formen entsprechen 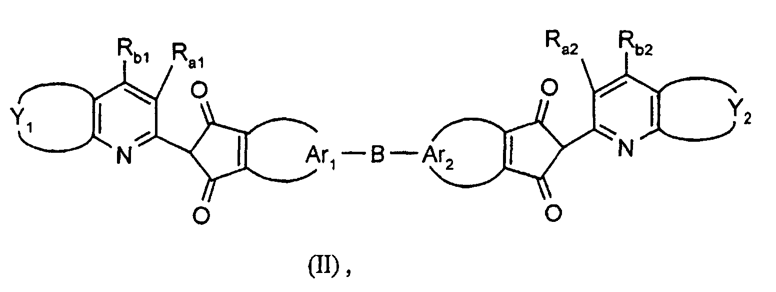 worin
Y₁ und Y₂ unabhängig voneinander den Rest eines gegebenenfalls substituierten Benzol- oder Naphthalinringes bilden,
Rₐ₁ und Rₐ₂ unabhängig voneinander H oder OH bedeuten und
R_{b1} und R_{b2} unabhängig voneinander H oder Halogen, insbesondere F, Br oder Cl bedeuten.

4. Verbindungen gemäß Anspruch 3, die der Formel (IIa) oder deren tautomeren Formen entsprechen 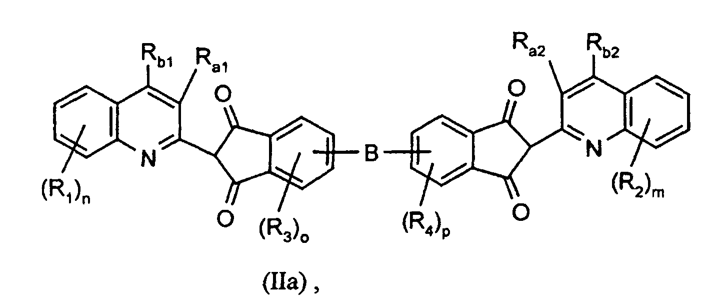 worin
n und m unabhängig voneinander für eine Zahl von 0 bis 4 stehen,
R₁ und R₂ unabhängig voneinander jeweils gleich oder verschieden sind und Halogen, insbesondere Cl und Br, -COOH, -COOR, wobei R für C₁-C₁₀-Alkyl, insbesondere Methyl und Ethyl, C₆-C₁₀-Aryl oder C₅-C₈-Cycloalkyl steht, und C₁-C₆-Alkyl, insbesondere Methyl bedeuten,
o und p unabhängig voneinander eine Zahl von 0 bis 2, insbesondere 0 oder 1 bedeuten,
R₃ und R₄ unabhängig voneinander jeweils gleich oder verschieden sind und C₁-C₆-Alkyl, insbesondere Methyl, Ethyl, n-Propyl, iso-Propyl, n-, sek- und tert.-Butyl, Halogen, insbesondere Cl und Br, Alkyl- und Arylcarbonyl, Alkyl- und Arylsulfonyl, Nitro, Aryl, insbesondere gegebenenfalls substituiertes Phenyl, Aryloxysulfonyl, insbesondere -SO₂OC₆H₅, Hydroxy, C₁-C₆-Alkoxy, wie Methoxy oder Benzyloxy, Aryloxy, wie Phenoxy, gegebenenfalls durch Alkyl oder Acyl substituiertes Amino wie NH₂, NHCOCH₃ und -N(C₂H₅)₂, gegebenenfalls durch Alkyl oder Aryl substituiertes Aminosulfonyl wie SO₂N(CH₃)₂ und SO₂NHCH₃, gegebenenfalls durch Alkyl substituiertes Carbonamid oder einen ankondensierten aromatischen, cycloaliphatischen oder heterocyclischen Ring bedeuten.

5. Verbindungen gemäß Anspruch 1, **dadurch gekennzeichnet, dass** B für einen Rest der Formeln 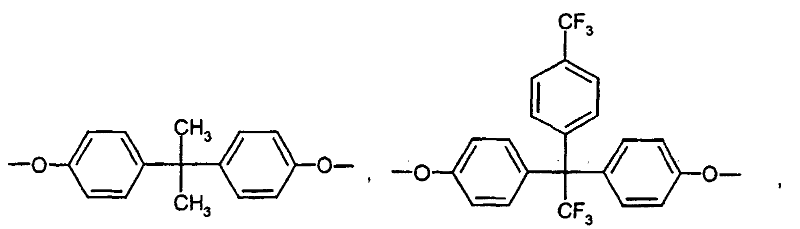 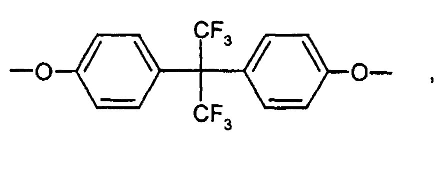
-O-CH₂-CH₂-O- , 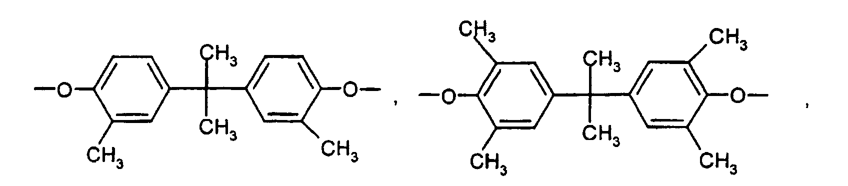 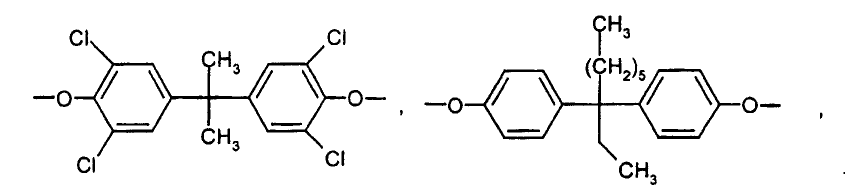 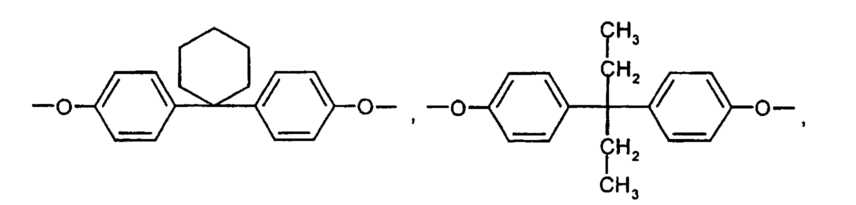 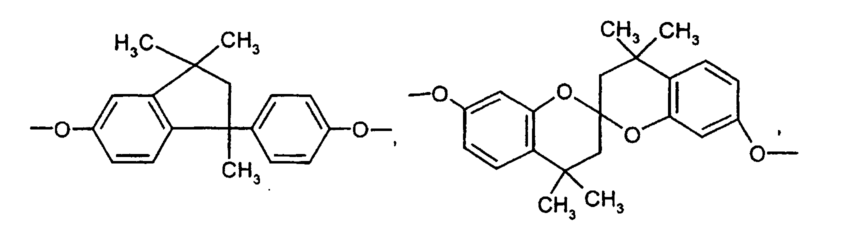 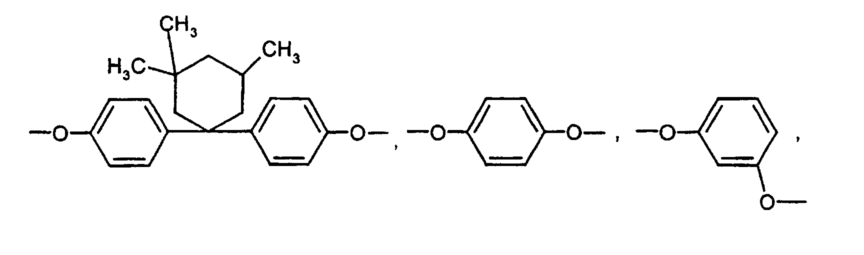 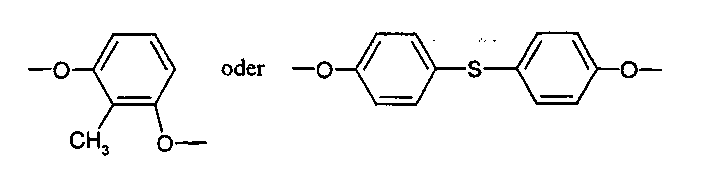
sowie die entsprechenden Dithio-Verbindungen (T₁ und T₂ = S), steht.

6. Verbindungen gemäß Anspruch 3, **dadurch gekennzeichnet, dass**
Y₁ = Y₂
Rₐ₁ = Rₐ₂
R_{b1} = R_{b2} und
Ar₁ = Ar₂ bedeuten.

7. Verbindungen gemäß Anspruch 4, **dadurch gekennzeichnet, dass**
n = m,
R₁ = R₂,
Rₐ₁ = Rₐ₂,
R_{b1} = R_{b2}
o = p und
R₃ = R₄ bedeutet,
wobei insbesondere
n, m, o und p für 0 stehen.

8. Verbindungen gemäß Anspruch 4, die der Formel (IIb) oder deren tautomeren Formen entsprechen

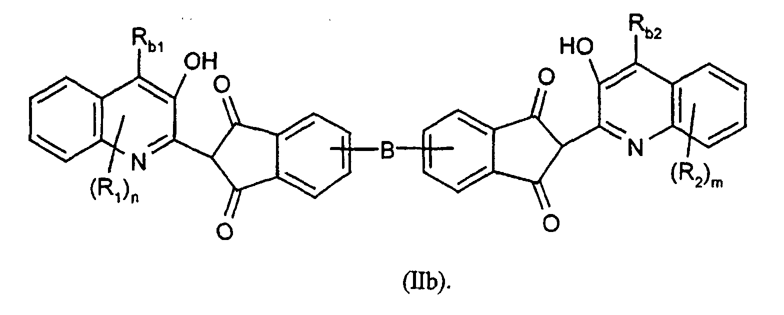

9. Verbindungen gemäß Anspruch 1, die der Formel (III) oder deren tautomeren Formen entsprechen 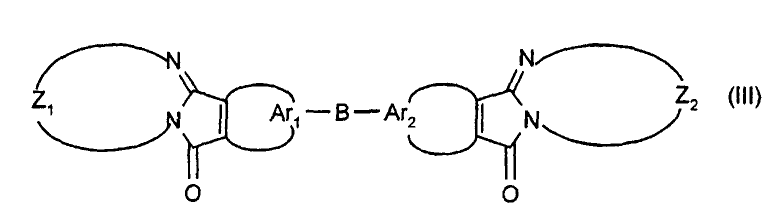 worin
Z₁ und Z₂ unabhängig voneinander gegebenenfalls substituiertes ortho-Phenylen, ortho-Naphthylen, peri-(1,8)-Naphthylen oder Arylen aus mehr als zwei miteinander kondensierten Benzolringen bedeuten, wobei die Arylreste, die mehr als zwei miteinander kondensierte Benzolringe aufweisen, in ortho- oder entsprechend einer peri-Stellung im Naphthalin verbrückt sind.

10. Verbindungen gemäß Anspruch 9, worin die Reste Z₁ und Z₂ unabhängig voneinander einem gegebenenfalls substituierten peri-(1,8)-Naphthylen entsprechen.

11. Verbindungen gemäß Anspruch 9, die der Formel (IIIa) entsprechen 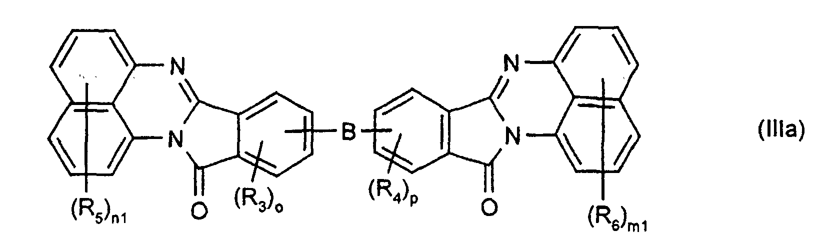 worin
n₁ und m₁ unabhängig voneinander für eine Zahl von 0 bis 4 stehen,
R₅ und R₆ unabhängig voneinander jeweils gleich oder verschieden sind und C₁-C₆-Alkyl, Halogen, Nitro, Aryl, Aryloxysulfonyl, Hydroxy, C₁-C₆-Alkoxy, Aryloxy, gegebenenfalls durch Alkyl oder Acyl substituiertes Amino, gegebenenfalls durch Alkyl oder Aryl substituiertes Aminosulfonyl, gegebenenfalls durch Alkyl substituiertes Carbonamid oder einen ankondensierten aromatischen, cycloaliphatischen oder heterocyclischen Ring bedeuten, insbesondere für Halogen und C₁-C₆-Alkyl, stehen,
o und p unabhängig voneinander eine Zahl von 0 bis 2, insbesondere 0 oder 1, bedeuten,
R₃ und R₄ unabhängig voneinander jeweils gleich oder verschieden sind und C₁-C₆-Alkyl, Halogen, Nitro, Aryl, Aryloxysulfonyl, Hydroxy, C₁-C₆-Alkoxy, Aryloxy, gegebenenfalls durch Alkyl oder Acyl substituiertes Amino, gegebenenfalls durch Alkyl oder Aryl substituiertes Aminosulfonyl gegebenenfalls durch Alkyl substituiertes Carbonamid oder einen ankondensierten aromatischen, cycloaliphatischen oder heterocyclischen Ring bedeuten, inbesondere Halogen, NO₂, -NH₂, -NH-Acyl oder -NH-Alkyl bedeuten.

12. Verbindungen gemäß Anspruch 9,
worin
Z₁ = Z₂ und
Ar₁ = Ar₂ bedeuten.

13. Verbindungen gemäß Anspruch 11, worin
n₁ = m₁,
R₅ = R₆,
o = p und
R₃ = R₄ bedeuten,
wobei insbesondere
n₁, m₁, o und p für 0 stehen.

14. Verbindungen gemäß Anspruch 11, die der Formel (IIIb) oder (IIIc) entsprechen

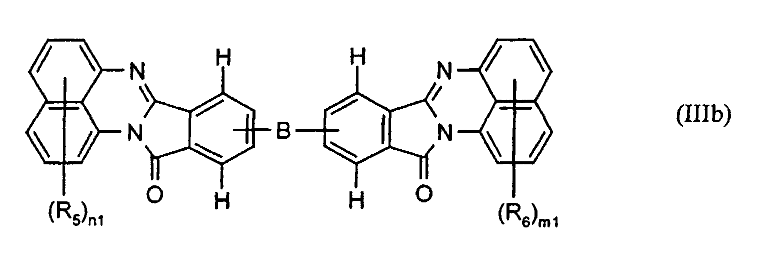

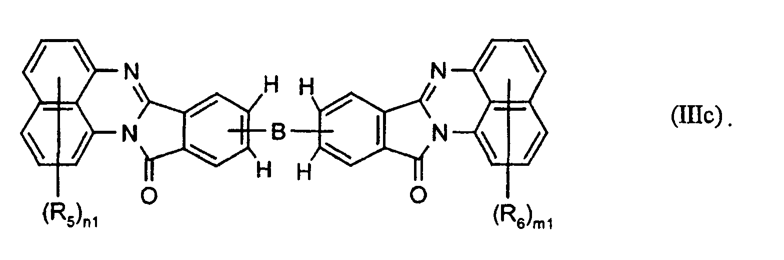

15. Verbindungen gemäß Anspruch 1, die der Formel (IV) oder deren tautomeren Formen entsprechen

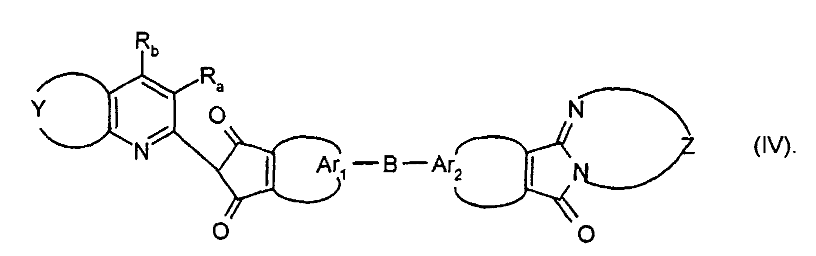

16. Verbindungen gemäß Anspruch 15, die der Formel (IVa) oder deren tautomeren Formen entsprechen 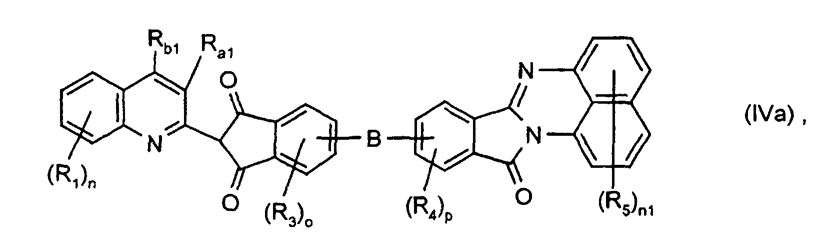 worin
Rₐ₁ H oder OH bedeutet, und
R_{b1} H oder Halogen, insbesondere F, Br oder Cl bedeutet,
n und n₁ unabhängig voneinander für eine Zahl von 0 bis 4 stehen,
R₁ Halogen, insbesondere Cl und Br, -COOH, -COOR, wobei R für C₁-C₁₀-Alkyl, insbesondere Methyl und Ethyl, C₆-C₁₀-Aryl oder C₅-C₈-Cycloalkyl steht, oder C₁-C₆-Alkyl, insbesondere Methyl bedeuten,
o und p unabhängig eine Zahl von 0 bis 2, insbesondere 0 oder 1 bedeuten,
R₃ und R₄ unabhängig voneinander jeweils gleich oder verschieden sind und C₁-C₆-Alkyl, insbesondere Methyl, Ethyl, n-Propyl, iso-Propyl, n-, sek- und tert.-Butyl, Halogen, insbesondere Cl und Br, Alkyl- und Arylcarbonyl, Alkyl- und Arylsulfonyl, Nitro, Aryl, insbesondere gegebenenfalls substituiertes Phenyl, Aryloxysulfonyl, insbesondere -SO₂OC₆H₅, Hydroxy, C₁-C₆-Alkoxy, wie Methoxy oder Benzyloxy, Aryloxy, wie Phenoxy gegebenenfalls durch Alkyl oder Acyl substituiertes Amino wie NH₂, NHCOCH₃ und -N(C₂H₅)₂, gegebenenfalls durch Alkyl oder Aryl substituiertes Aminosulfonyl wie SO₂N(CH₃)₂ und SO₂NHCH₃ gegebenenfalls durch Alkyl substituiertes Carbonamid oder einen ankondensierten aromatischen, cycloaliphatischen oder heterocyclischen Ring bedeuten, und
R₅ C₁-C₆-Alkyl, Halogen, Nitro, Aryl, Aryloxysulfonyl, Hydroxy, C₁-C₆-Alkoxy, Aryloxy, gegebenenfalls durch Alkyl oder Acyl substituiertes Amino, gegebenenfalls durch Alkyl oder Aryl substituiertes Aminosulfonyl gegebenenfalls durch Alkyl substituiertes Carbonamid oder einen ankondensierten aromatischen, cycloaliphatischen oder heterocyclischen Ring bedeuten, insbesondere für Halogen und C₁-C₆-Alkyl steht.

17. Verbindungen gemäß Anspruch 16, die der Formel (IVb) oder deren tautomeren Formen entsprechen

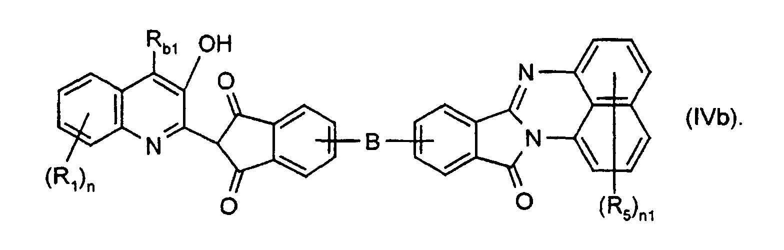

18. Verfahren zur Herstellung der erfindungsgemäßen Verbindungen der Formel (I) gemäß Anspruch 1, das **dadurch gekennzeichnet ist, dass** man Tetracarbonsäuren oder deren Anhydride der Formel (V) 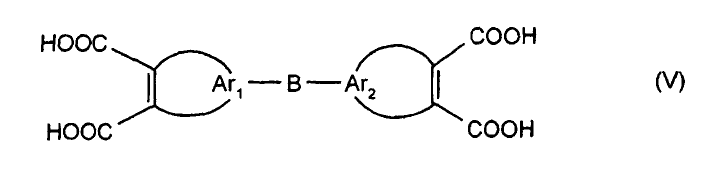 mit einer oder mehreren Verbindungen der Formeln (VI) und/oder (VII) 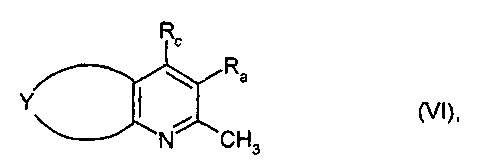 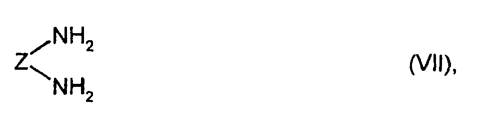 worin
R_{c} H, COOH oder Halogen, insbesondere F, Br und Cl bedeuten, insbesondere für H oder COOH steht und
Ar₁, Ar₂, B, Y, Z und Rₐ die in Anspruch 1 angegebene Bedeutung besitzen,
kondensiert, wobei die Summe der Verbindungen der Formeln (VI) und (VII) zwei Mol-Äquivalenten, bezogen auf die Tetracarbonsäure (V), entspricht.

19. Verwendung einer Verbindung gemäß Anspruch 1 zum Massefärben von Kunststoffen.

20. Verwendung gemäß Anspruch 19, **dadurch gekennzeichnet, dass** der Kunststoff ein Thermoplast ist.

21. Verwendung nach Anspruch 19, **dadurch gekennzeichnet, dass** der zu färbende Kunststoff ein Vinylpolymer, insbesondere Polystyrol, ein Polyester oder Acrylnitril-Butadien-Styrol (ABS), Styrol-Acrylnitril (SAN), Polymethylmethacrylat (PMMA) oder Polycarbonat ist.

22. Kunststoffe, gefärbt mit wenigstens einer Verbindung gemäß Anspruch 1.

## Claims

1. Compounds of the general formula (I) or tautomeric forms thereof 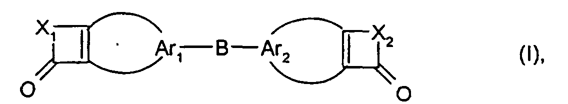 where
Ar1 and Ar2 are independently radicals needed to complete optionally substituted carbocyclic aromatics,
B is a radical of the formula -T1-W-T2-, where
T1 and T2 are independently O or S and
W is alkylene, especially C1-C6-alkylene, C6-C10-arylene, especially phenylene or cycloalkylene, which are each optionally substituted or is the radical of the formula (a) 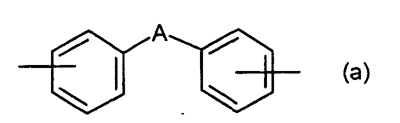 where the phenyl rings are optionally substituted and
A is a radical of the formula O, S, SO, SO2 or CO, optionally substituted alkylene, or optionally substituted cycloalkylene, said alkylene or cycloalkylene being attached to the adjacent phenyl rings itself or else via its substitutents, or
W is a radical of the formulae
-(CH₂)ₛ-O-(CH₂)ₜ-, -(CH₂)ₛ-S-(CH₂)ₜ-, 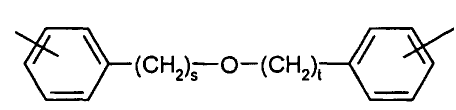
or 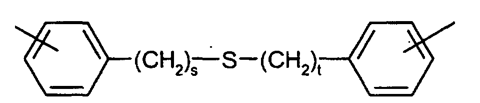 where
s and t are independently from 1 to 6,
the ends of the divalent radical B each being attached to an aromatic carbon atom of the two radicals Ar 1 and Ar2,
and
X1 and X2 are independently a radical of the formulae selected from the group consisting of 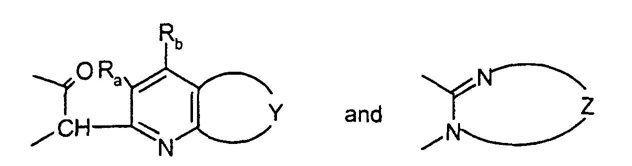
these each being located in the ring in such a way that the 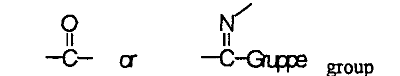 is adjacent to the C-C double bond,
where
Y is the radical of an optionally substituted benzene or naphthalene ring,
Z is optionally substituted ortho-phenylene, ortho-naphthylene, peri-(1,8)-naphthylene or arylene composed of more than two fused-together benzene rings, the aryl radicals which have more than two fused-together benzene rings being bridged ortho or in a manner corresponding to a peri position in naphthalene,
Ra is H or OH, and
Rb is H or halogen, especially F, Br or Cl.

2. Compounds according to Claim 1, **characterized in that** Ar1 and Ar2 are independently a radical needed to complete an optionally substituted benzene or naphthalene ring, especially an optionally substituted benzene ring.

3. Compounds according to Claim 1 which conform to the formula (II) or tautomeric forms thereof 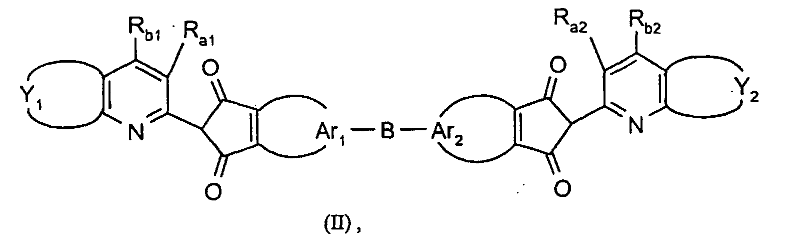 where
Y1 and Y2 are independently the radical of an optionally substituted benzene or naphthalene ring,
Ra1 and Ra2 are independently H or OH, and
Rb1 and Rb2 are independently H or halogen, especially F, Br or Cl.

4. Compounds according to Claim 3 which conform to the formula (IIa) or tautomeric forms thereof 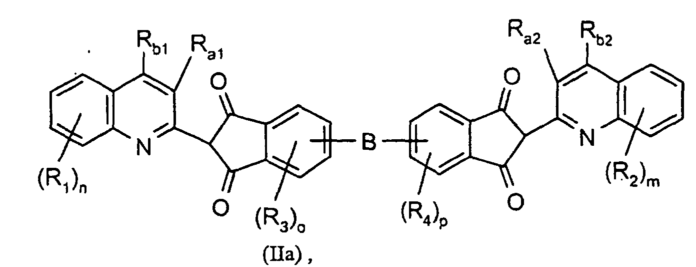 where
n and m are independently from 0 to 4,
R1 and R2 are each independently the same or different and represent halogen, especially Cl or Br, -COOH, -COOR, where R is C1-C10-alkyl, especially methyl or ethyl, C6-C10-aryl or C5-C8-cycloalkyl, or are C1-C6-alkyl, especially methyl,
o and p are independently from 0 to 2, especially 0 or 1,
R3 and R4 are each independently the same or different and represent C1-C6-alkyl, especially methyl, ethyl, n-propyl, iso-propyl, n-butyl, sec-butyl, tert-butyl, halogen, especially Cl or Br, alkylcarbonyl, arylcarbonyl, alkylsulphonyl, arylsulphonyl, nitro, aryl, especially optionally substituted phenyl, aryloxysulphonyl, especially -SO2OC6H5, hydroxyl, C1-C6-alkoxy, such as methoxy or benzyloxy, aryloxy, such as phenoxy, optionally alkyl- or acyl-substituted amino such as NH2, NHCOCH3 or -N(C2H5)2, optionally alkyl- or aryl-substituted aminosulphonyl such as SO2N(CH3)2 or SO2NHCH3, optionally alkyl-substituted carboxamide or a fused-on aromatic, cycloaliphatic or heterocyclic ring.

5. Compounds according to Claim 1, **characterized in that** B is a radical of the formulae 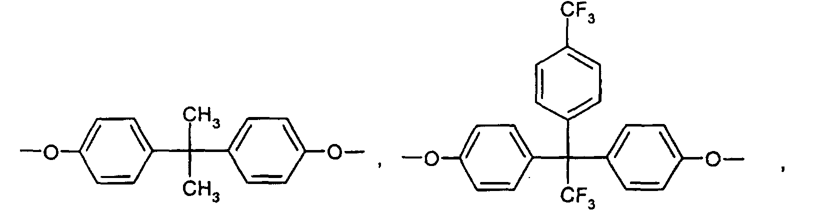 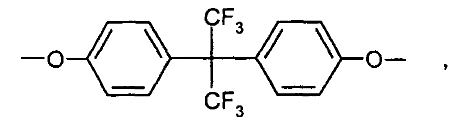
-O-CH₂-CH₂-O- , 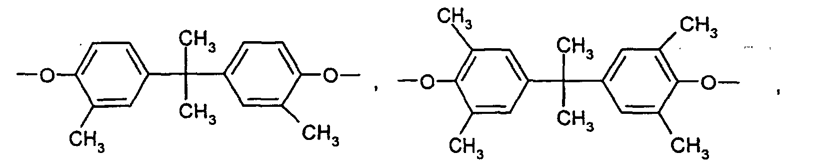 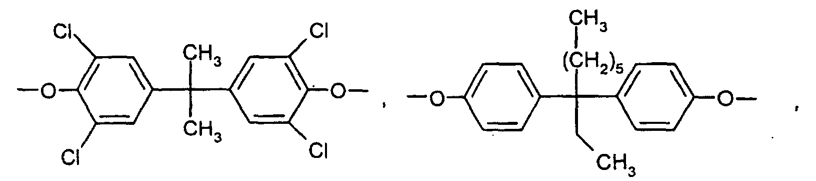 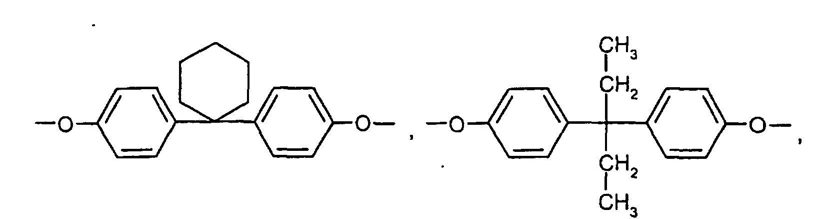 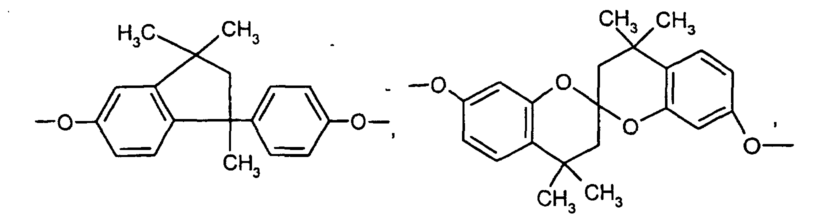 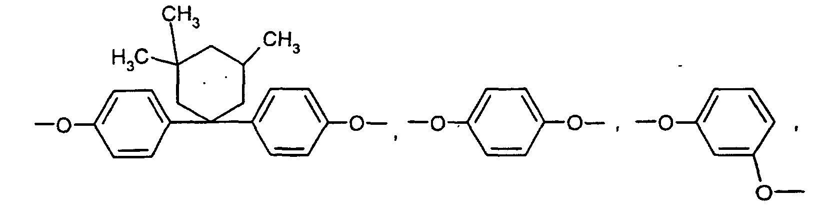 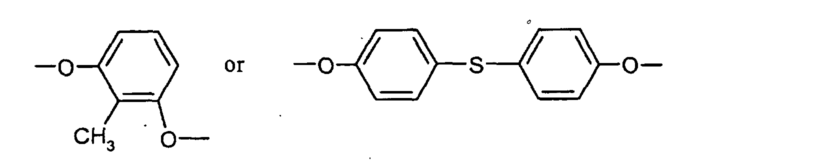
and also the corresponding dithio compounds (T1 and T2 = S).

6. Compounds according to Claim 3, **characterized in that**
Y1 = Y2
Ra1 = Ra2
Rb1 = Rb2 and
Ar1 = Ar2.

7. Compounds according to Claim 4, **characterized in that**
n = m,
R1 = R2,
Ra1 = Ra2,
Rb1 = Rb2
o = p and
R3 = R4,
where in particular
n, m, o and p are each 0.

8. Compounds according to Claim 4 which conform to the formula (IIb) or tautomeric forms thereof

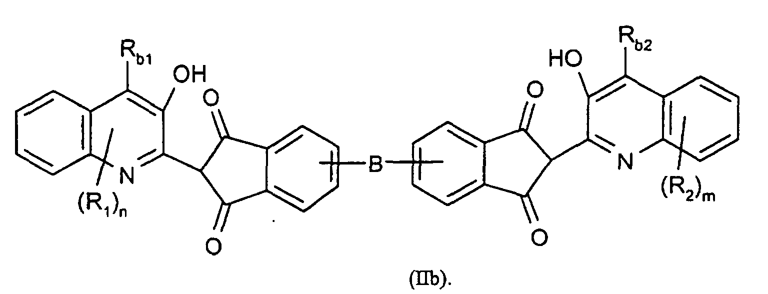

9. Compounds according to Claim 1 which conform to the formula (III) or tautomeric forms thereof 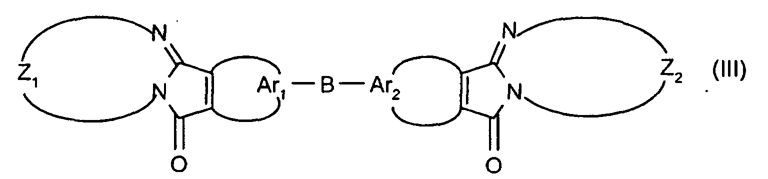 where
Z1 and Z2 are independently optionally substituted ortho-phenylene, ortho-naphthylene, peri-(1,8)-naphthylene or arylene composed of more than two fused-together benzene rings, the aryl radicals which have more than two fused-together benzene rings being bridged ortho or in a manner corresponding to a peri position in naphthalene.

10. Compounds according to Claim 9, wherein the radicals Z 1 and Z2 are independently an optionally substituted peri-(1,8)-naphthylene.

11. Compounds according to Claim 9 which conform to the formula (IIIa) 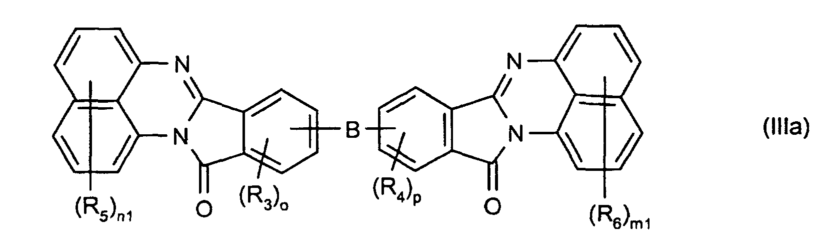 where
n1 and m1 are independently from 0 to 4,
R5 and R6 are each independently the same or different and represent C1-C6-alkyl, halogen, nitro, aryl, aryloxysulphonyl, hydroxyl, C1-C6-alkoxy, aryloxy, optionally alkyl- or acyl-substituted amino, optionally alkyl- or aryl-substituted aminosulphonyl, optionally alkyl-substituted carboxamide or a fused-on aromatic, cycloaliphatic or heterocyclic ring, especially halogen or C1-C6-alkyl,
o and p are independently from 0 to 2, especially 0 or 1,
R3 and R4 are each independently the same or different and represent C1-C6-alkyl, halogen, nitro, aryl, aryloxysulphonyl, hydroxyl, C1-C6-alkoxy, aryloxy, optionally alkyl- or acyl-substituted amino, optionally alkyl- or aryl-substituted aminosulphonyl, optionally alkyl-substituted carboxamide or a fused-on aromatic, cycloaliphatic or heterocyclic ring, especially halogen, NO2, -NH2, -NH-acyl or -NH-alkyl.

12. Compounds according to Claim 9,
where
Z1 = Z2 and
Ar1 = Ar2.

13. Compounds according to Claim 11, where
n1 = m1,
R5 = R6,
o = p and
R3 = R4,
where in particular
n1, m1, o and p are each 0.

14. Compounds according to Claim 11 which conform to the formula (IIIb) or (IIIc)

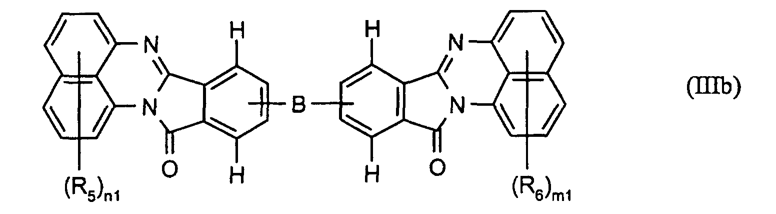

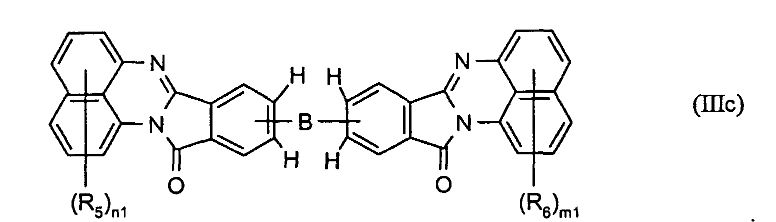

15. Compounds according to Claim 1 which conform to the formula (IV) or tautomeric forms thereof

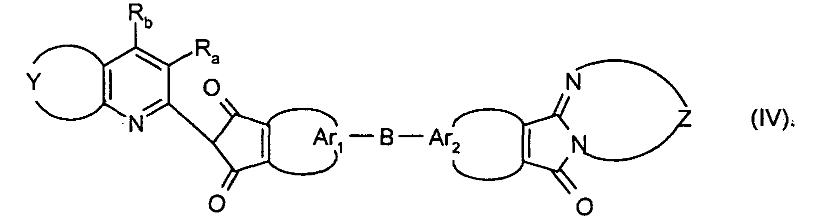

16. Compounds according to Claim 15 which conform to the formula (IVa) or tautomeric forms thereof 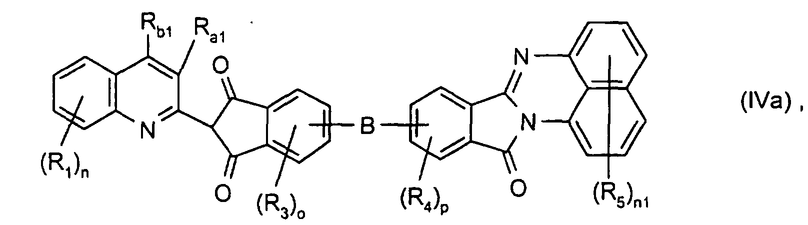 where
Ra1 is H or OH,
Rb1 is H or halogen, especially F, Br or Cl,
n and n1 are independently from 0 to 4,
each R1 is halogen, especially Cl or Br, -COOH, -COOR, where R is C1-C10-alkyl, especially methyl or ethyl, C6-C10-aryl or C5-C8-cycloalkyl, or is C1-C6-alkyl, especially methyl,
o and p are independently from 0 to 2, especially 0 or 1,
R3 and R4 are each independently the same or different and represent C1-C6-alkyl, especially methyl, ethyl, n-propyl, iso-propyl, n-butyl, sec-butyl, tert-butyl, halogen, especially Cl or Br, alkylcarbonyl, arylcarbonyl, alkylsulphonyl, arylsulphonyl, nitro, aryl, especially optionally substituted phenyl, aryloxysulphonyl, especially -SO2OC6H5, hydroxyl, C1-C6-alkoxy, such as methoxy or benzyloxy, aryloxy, such as phenoxy, optionally alkyl- or acyl-substituted amino such as NH2, NHCOCH3 or -N(C2H5)2, optionally alkyl- or aryl-substituted aminosulphonyl such as SO2N(CH3)2 or SO2NHCH3, optionally alkyl-substituted carboxamide or a fused-on aromatic, cycloaliphatic or heterocyclic ring, and
each R5 is C1-C6-alkyl, halogen, nitro, aryl, aryloxysulphonyl, hydroxyl, C1-C6-alkoxy, aryloxy, optionally alkyl- or acyl-substituted amino, optionally alkyl- or aryl-substituted aminosulphonyl, optionally alkyl-substituted carboxamide or a fused-on aromatic, cycloaliphatic or heterocyclic ring, especially halogen or C1-C6-alkyl.

17. Compounds according to Claim 16 which conform to the formula (IVb) or tautomeric forms thereof

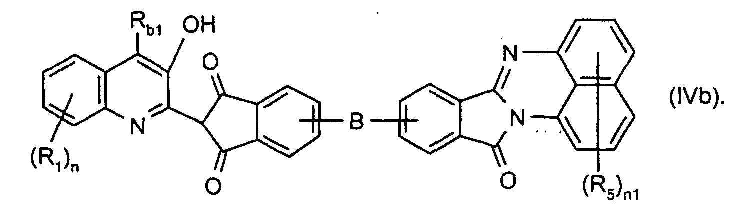

18. Process for preparing the compounds of the formula (I) according to the invention as set forth in Claim 1, **characterized in that** tetracarboxylic acids or anhydrides of the formula (V) 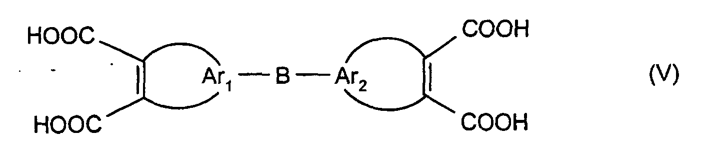 are condensed with one or more compounds of the formulae (VI) and/or (VII) 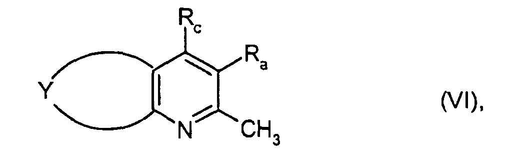 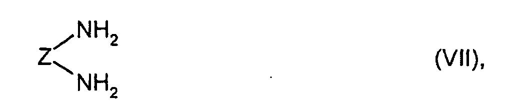 where
Rc is H, COOH or halogen, especially F, Br or Cl, especially H or COOH, and
Ar1, Ar2, B, Y, Z and Ra are each as defined in Claim 1,
the sum total of the compounds of the formulae (VI) and (VII) being equal to two mole equivalents, based on tetracarboxylic acid (V).

19. Use of a compound according to Claim 1 for mass coloration of plastics.

20. Use according to Claim 19, **characterized in that** the plastic is a thermoplastic.

21. Use according to Claim 19, **characterized in that** the plastic to be coloured is a vinyl polymer, especially polystyrene, a polyester or acrylonitrile-butadiene-styrene (ABS), styrene-acrylonitrile (SAN), polymethyl methacrylate (PMMA) or polycarbonate.

22. Plastics coloured with at least one compound according to Claim 1.

## Revendications

1. Composés de formule générale (I) ou ses formes tautomères 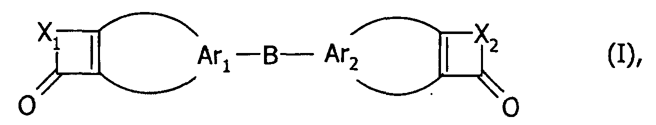 où
Ar₁ et Ar₂ représentent indépendamment l'un de l'autre des groupements pour compléter des aromatiques carbocycliques éventuellement substitués,
B représente un groupement de formule -T₁-W-T₂-, où
T₁ et T₂ représentent indépendamment l'un de l'autre O ou S et
W représente alkylène, en particulier C₁-C₆-alkylène, C₆-C₁₀-arylène, en particulier phénylène ou cycloalkylène, qui, dans chaque cas, sont éventuellement substitués, ou le groupement de formule (a) 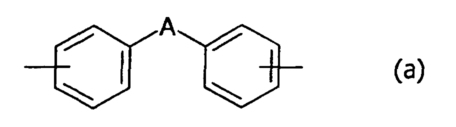 où les cycles phényle sont éventuellement substitués
et
A représente un groupement de formule O, S, SO, SO₂, CO, alkylène éventuellement substitué, cycloalkylène éventuellement substitué, où l'alkylène ou le cycloalkylène peut dans chaque cas être lié par lui-même ou encore par le biais de l'un de ses substituants aux cycles phényle voisins, ou
W représente un groupement des formules
-(CH₂)ₛ-O-(CH₂)ₜ-, -(CH₂)ₛ-S-(CH₂)ₜ- , 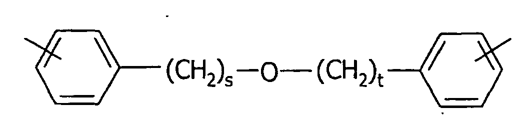
ou 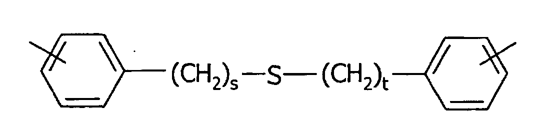 où
s et t représentent indépendamment l'un de l'autre un nombre de 1 à 6,
où les extrémités du groupement divalent B sont liées dans chaque cas à un atome de carbone aromatique des deux groupements Ar₁ et Ar₂, et
X₁ et X₂ représentent indépendamment l'un de l'autre un groupement des formules choisies dans le groupe 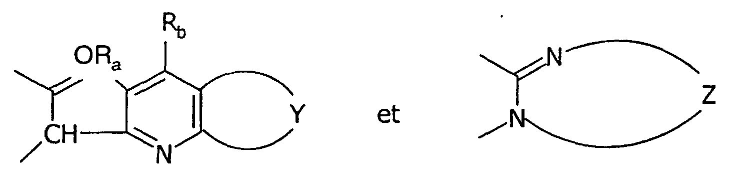 où ils sont disposés dans chaque cas dans le cycle de telle manière que le groupe 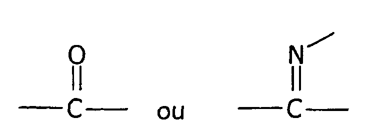 est voisin de la double liaison C-C,
où
Y forme le reste d'un cycle benzène ou naphtalène éventuellement substitué,
Z représente ortho-phénylène, ortho-naphtylène, péri-(1,8)-naphtylène ou arylène éventuellement substitué constitué par plus de deux cycles benzéniques condensés entre eux, où les groupements aryle comportant plus de deux cycles benzéniques condensés entre eux sont pontés en position ortho ou d'une manière correspondant à une position péri dans le naphtalène,
Rₐ représente H ou OH et
R_{b} représente H ou halogène, en particulier F, Br et CI.

2. Composés selon la revendication 1, **caractérisés en ce que** Ar₁ et Ar₂ représentent indépendamment l'un de l'autre un groupement pour compléter un cycle benzénique ou naphtalène éventuellement substitué, en particulier un cycle benzénique éventuellement substitué.

3. Composés selon la revendication 1 qui correspondent à la formule (II) ou ses formes tautomères 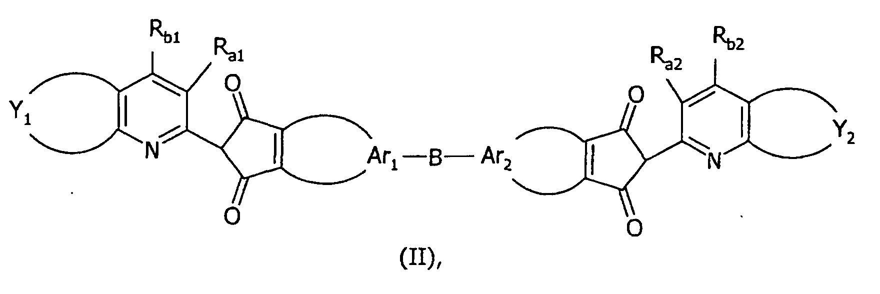 où
Y₁ et Y₂ forment indépendamment l'un de l'autre le reste d'un cycle benzénique ou naphtalène éventuellement substitué,
Rₐ₁ et Rₐ₂ représentent indépendamment l'un de l'autre H ou OH,
R_{b1} et R_{b2} représentent indépendamment l'un de l'autre H ou halogène, en particulier F, Br ou Cl.

4. Composés selon la revendication 3 qui correspondent à la formule (IIa) ou ses formes tautomères 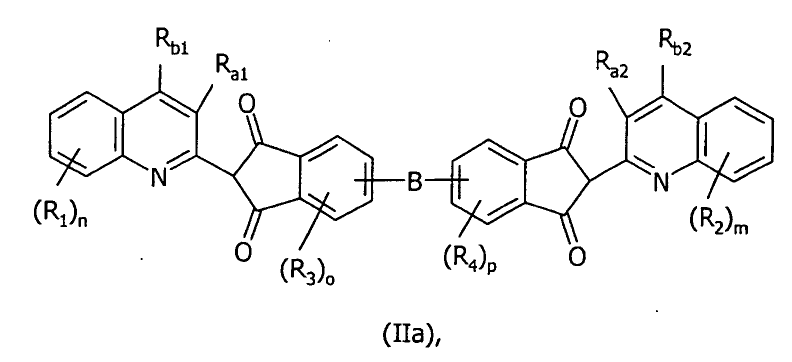 où
n et m représentent indépendamment l'un de l'autre un nombre de 0 à 4,
R₁ et R₂, indépendamment l'un de l'autre, sont identiques ou différents et représentent halogène, en particulier Cl et Br, -COOH, -COOR, où R représente C₁-C₁₀-alkyle, en particulier méthyle et éthyle, C₆-C₁₀-aryle ou C₅-C₈-cycloalkyle, et C₁-C₆-alkyle, en particulier méthyle,
o et p représentent indépendamment l'un de l'autre un nombre de 0 à 2, en particulier 0 ou 1,
R₃ et R₄, indépendamment l'un de l'autre, sont identiques ou différents et représentent C₁-C₆-alkyle, en particulier méthyle, éthyle, n-propyle, isopropyle, n-, sec- et tert-butyle, halogène, en particulier Cl et Br, alkyl- et arylcarbonyle, alkyl- et arylsulfonyle, nitro, aryle, en particulier phényle éventuellement substitué, aryloxysulfonyle, en particulier -SO₂OC₆H₅, hydroxyle, C₁-C₆-alcoxy, comme éthoxy ou benzyloxy, aryloxy, comme phénoxy, amino éventuellement substitué par alkyle ou acyle comme NH₂, NHCOCH₃ et -N(C₂H₅)₂, aminosulfonyle éventuellement substitué par alkyle ou aryle comme SO₂N(CH₃)₂ et SO₂NHCH₃, carbonamide éventuellement substitué par alkyle ou un cycle aromatique, cycloaliphatique ou hétérocyclique condensé.

5. Composés selon la revendication 1, **caractérisés en ce que** B représente un groupement des formules 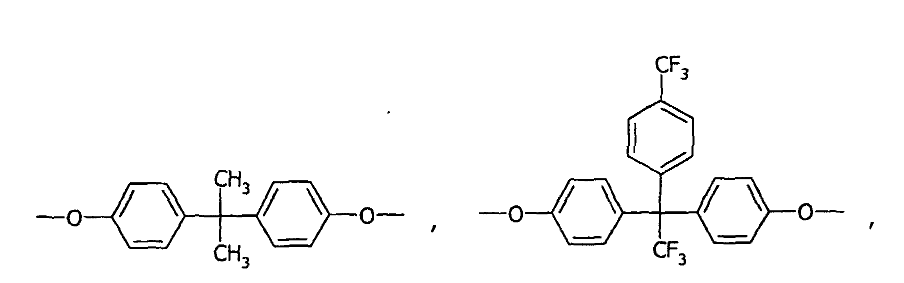 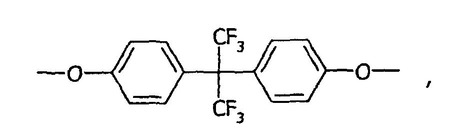
-O-CH₂-CH₂-O- , 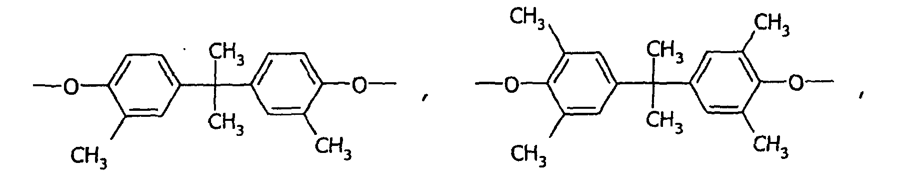 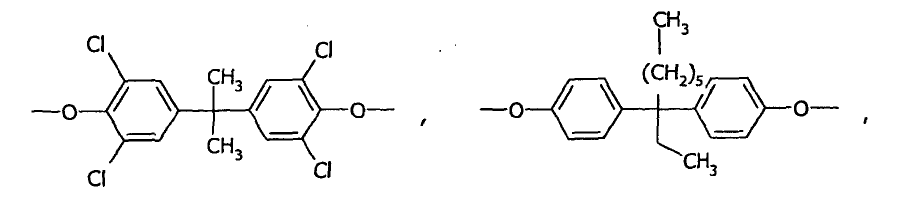 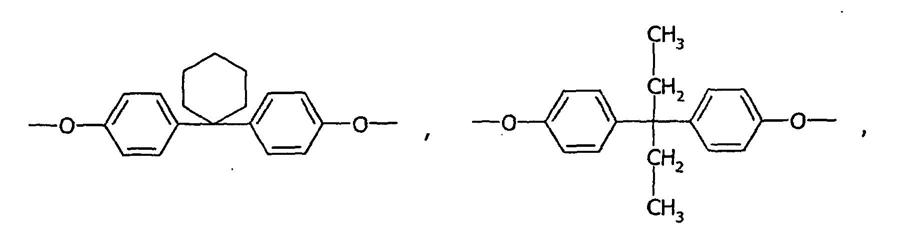 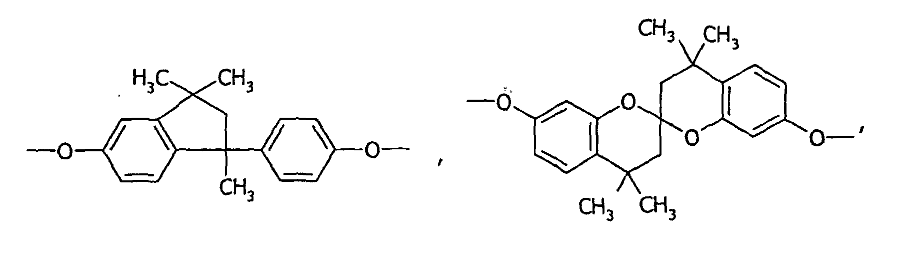 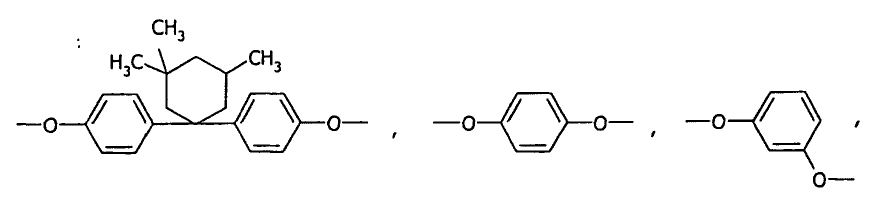 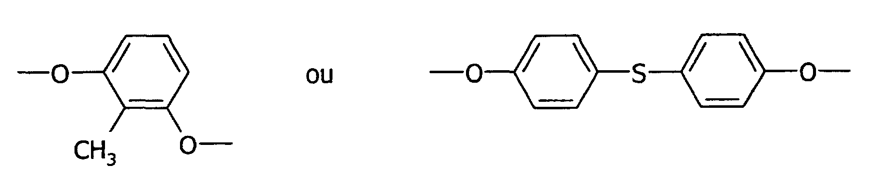
ainsi que les composés dithio correspondants (T₁ et T₂ = S).

6. Composés selon la revendication 3, **caractérisés en ce que**
Y₁ = Y₂
Rₐ₁ = Rₐ₂
R_{b1} = R_{b2} et
Ar₁ = Ar₂.

7. Composés selon la revendication 4, **caractérisés en ce que**
n = m,
R₁ = R₂,
Rₐ₁ = Rₐ₂,
R_{b1} = R_{b2},
o = p et
R₃ = R₄,
où en particulier
n, m, o et p représentent 0.

8. Composés selon la revendication 4 qui correspondent à la formule (IIb) ou ses formes tautomères

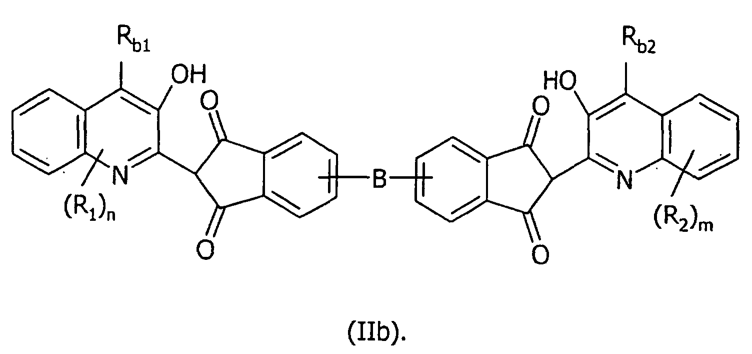

9. Composés selon la revendication 1 qui correspondent à la formule (III) ou ses formes tautomères 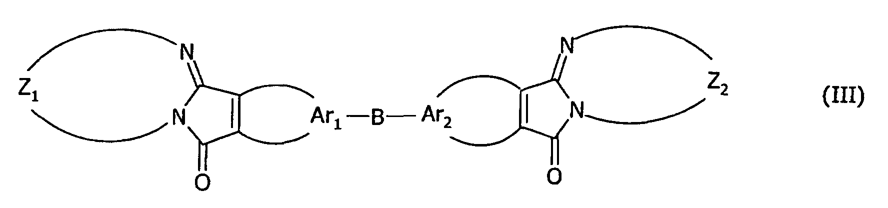 où
Z₁ et Z₂ représentent indépendamment l'un de l'autre ortho-phénylène, ortho-naphtylène, péri-(1,8)-naphtylène ou arylène éventuellement substitué constitué par plus de deux cycles benzéniques condensés entre eux, où les groupements alkyle qui comportent plus de deux cycles benzéniques condensés entre eux sont pontés en position ortho ou d'une manière correspondant à une position péri dans le naphtalène.

10. Composés selon la revendication 9 où les groupements Z₁ et Z₂ correspondent indépendamment l'un de l'autre à un péri-(1,8)-naphtylène éventuellement substitué.

11. Composés selon la revendication 9 qui correspondent à la formule (IIIa) 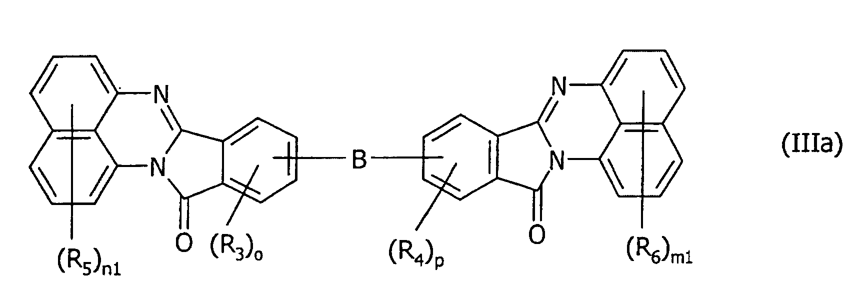 où
n₁ et m₁ représentent indépendamment l'un de l'autre un nombre de 0 à 4,
R₅ et R₆, indépendamment l'un de l'autre, sont identiques ou différents et représentent C₁-C₆-alkyle, halogène, nitro, aryle, aryloxysulfonyle, hydroxyle, C₁-C₆-alcoxy, aryloxy, amino éventuellement substitué par alkyle ou acyle, aminosulfonyle éventuellement substitué par alkyle ou aryle, carbonamide éventuellement substitué par alkyle ou un cycle aromatique, cycloaliphatique ou hétérocyclique condensé, en particulier halogène et C₁-C₆-alkyle,
o et p représentent indépendamment l'un de l'autre un nombre de 0 à 2, en particulier 0 ou 1,
R₃ et R₄, indépendamment l'un de l'autre, sont identiques ou différents et représentent C₁-C₆-alkyle, halogène, nitro, aryle, aryloxysulfonyle, hydroxyle, C₁-C₆-alcoxy, aryloxy, amino éventuellement substitué par alkyle ou acyle, aminosulfonyle éventuellement substitué par alkyle ou aryle, carbonamide éventuellement substitué par alkyle ou un cycle aromatique, cycloaliphatique ou hétérocyclique condensé, en particulier halogène, NO₂, -NH₂, -NH-acyle ou -NH-alkyle.

12. Composés selon la revendication 9 où
Z₁ = Z₂ et
Ar₁ = Ar₂.

13. Composés selon la revendication 11, où
n₁ = m₁,
R₅ = R₆,
o = p et
R₃ = R₄,
où en particulier
n₁, m₁, o et p représentent 0.

14. Composés selon la revendication 11 qui correspondent à la formule (IIIb) ou (IIIc)

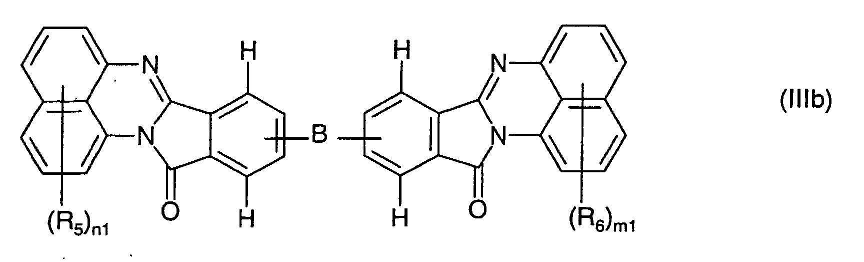

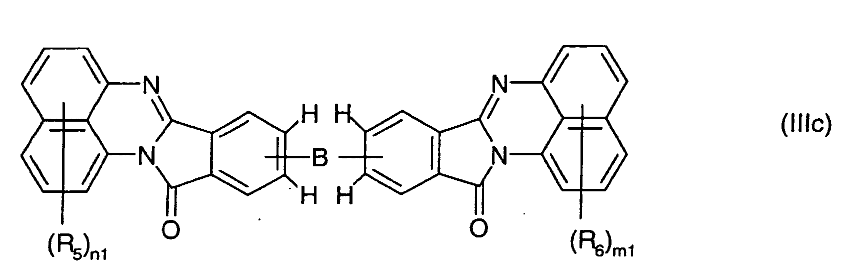

15. Composés selon la revendication 1 qui correspondent à la formule (IV) ou ses formes tautomères

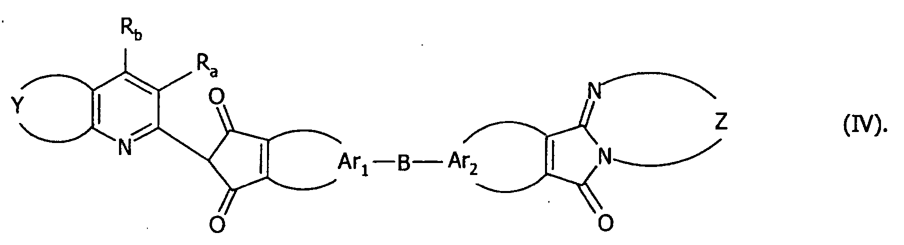

16. Composés selon la revendication 15 qui correspondent à la formule (Na) ou ses formes tautomères 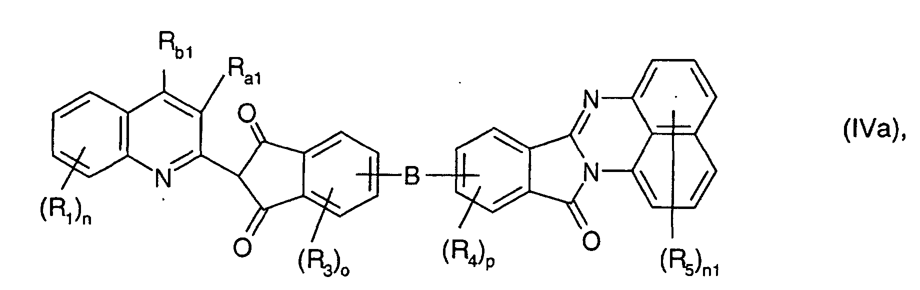 où
Rₐ₁ représente H ou OH, et
R_{b1} représente H ou halogène, en particulier F, Br ou Cl,
n et n₁ représentent indépendamment l'un de l'autre un nombre de 0 à 4,
R₁ représente halogène, en particulier Cl et Br, -COOH, -COOR, où R représente C₁-C₁₀-alkyle, en particulier méthyle et éthyle, C₆-C₁₀-aryle ou C₅-C₈-cycloalkyle, ou C₁-C₆-alkyle, en particulier méthyle,
o et p représentent indépendamment un nombre de 0 à 2, en particulier 0 ou 1,
R₃ et R₄, indépendamment l'un de l'autre, sont identiques ou différents et représentent C₁-C₆-alkyle, en particulier méthyle, éthyle, n-propyle, isopropyle, n-, sec- et tert-butyle, halogène, en particulier CI et Br, alkyl- et arylcarbonyle, alkyl- et arylsulfonyle, nitro, aryle, en particulier phényle éventuellement substitué, aryloxysulfonyle, en particulier -SO₂OC₆H₅, hydroxyle, C₁-C₆-alcoxy, comme méthoxy ou benzyloxy, aryloxy, comme phénoxy, amino éventuellement substitué par alkyle ou acyle comme NH₂, NHCOCH₃ et -N(C₂H₅)₂, aminosulfonyle éventuellement substitué par alkyle ou aryle comme SO₂N(CH₃)₂ et SO₂NHCH₃, carbonamide éventuellement substitué par alkyle ou un cycle aromatique, cycloaliphatique ou hétérocyclique condensé, et
R₅ représente C₁-C₆-alkyle, halogène, nitro, aryle, aryloxysulfonyle, hydroxyle, C₁-C₆-alcoxy, aryloxy, amino éventuellement substitué par alkyle ou acyle, aminosulfonyle éventuellement substitué par alkyle ou aryle, carbonamide éventuellement substitué par alkyle ou un cycle aromatique, cycloaliphatique ou hétérocyclique condensé, en particulier halogène et C₁-C₆-alkyle.

17. Composés selon la revendication 16 qui correspondent à la formule (Nb) ou ses formes tautomères

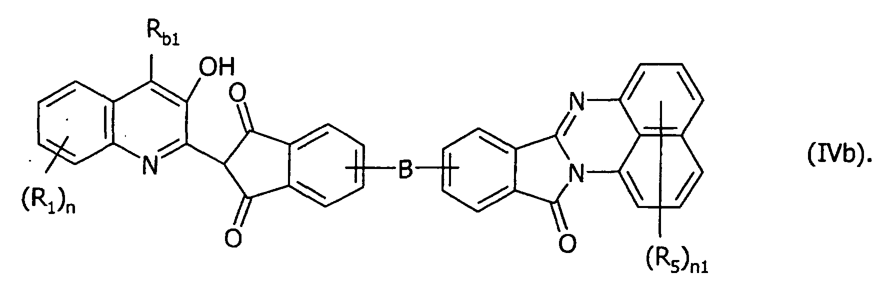

18. Procédé de préparation des composés selon l'invention de formule (I) selon la revendication 1, qui est **caractérisé en ce que** l'on condense des acides tétracarboxyliques ou leurs anhydrides de formule (V) 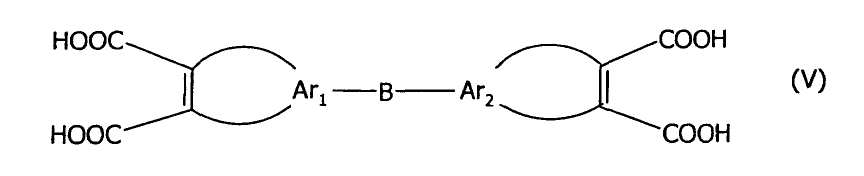 avec un ou plusieurs composés des formules (VI) et/ou (VII) 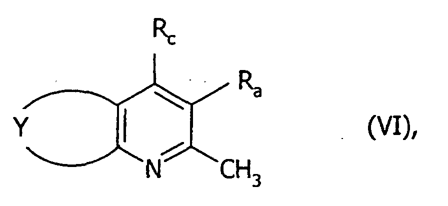 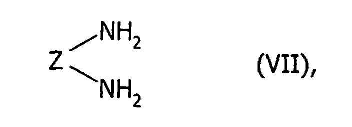
R_{c} représente H, COOH ou halogène, en particulier F, Br et Cl, en particulier H ou COOH et
Ar₁, Ar₂, B, Y, Z et Rₐ possèdent la signification indiquée dans la revendication 1,
où la somme des composés des formules (VI) et (VII) correspond à deux équivalents molaires par rapport à l'acide tétracarboxylique (V).

19. Utilisation d'un composé selon la revendication 1 pour la coloration de matières plastiques dans la masse.

20. Utilisation selon la revendication 19, **caractérisée en ce que** la matière plastique est une matière thermoplastique.

21. Utilisation selon la revendication 19, **caractérisée en ce que** la matière plastique à colorer est un polymère vinylique, en particulier le polystyrène, un polyester ou l'acrylonitrile-butadiène-styrène (ABS), le styrène-acrylonitrile (SAN), le poly(méthacrylate de méthyle) (PMMA) ou le polycarbonate.

22. Matières plastiques colorées avec au moins un composé selon la revendication 1.
